# EUROPEAN PATENT SPECIFICATION

(11) **EP 4 003 359 B1**
(45) Date of publication and mention of the grant of the patent: **23.07.2025**
(21) Application number: 21710916.4
(22) Date of filing: 05.03.2021
(51) Int. Cl.: A61K 31/5377, A61K 31/427, A61K 31/706, A61K 31/4015, A61K 31/4418, A61K 31/4433, A61K 31/4706, A61K 31/472, A61K 31/4965, A61K 31/513, A61K 31/635, A61K 31/7068, A61K 38/05, A61K 9/00, A61K 45/06, A61P 31/14

(54) **ORAL COBICISTAT FOR THE PREVENTION AND/OR TREATMENT OF SARS-COV-2, SARS-COV AND/OR MERS-COV INFECTIONS**
ORALER COBICISTAT ZUR VORBEUGUNG UND/ODER BEHANDLUNG VON INFEKTIONEN MIT SARS-COV-2, SARS-COV UND/ODER MERS-COV
COBICISTAT ORAL POUR LA PRÉVENTION ET/OU LE TRAITEMENT DES INFECTIONS CAUSÉES PAR LE SARS-COV-2, LE SARS-COV ET/OU LE MERS-COV

(30) Priority: 18.06.2020 US 202063040758 P
(43) Date of publication of application: 01.06.2022
(73) Proprietor: Universität Heidelberg, 69117 Heidelberg (DE)
(72) Inventor: FARES, Mohamed, Omar, Elfayoum (EG); TOLBA, Mahmoud, Mostafa, Elfayoum (EG); AYOUB, Ahmed, Taha, New Cairo (EG); LUSIC, Marina, 69126 Heidelberg (DE); SHYTAJ, Iart, Luca, 04038-033 São Paulo, SP (BR); SAVARINO, Andrea, 10048 Vinovo (IT); DIAZ, Ricardo, Sobhie, 06429-245 Barueri, SP (BR)
(74) Representative: Engelhard, Markus
(86) International application number: PCT/EP2021/055621
(87) International publication number: WO 2021/254667

(56) References cited:
- GALLUCCI LARA ET AL: "Broad-spectrum antiviral activity of two structurally analogous CYP3A inhibitors against pathogenic human coronaviruses in vitro", ANTIVIRAL RESEARCH, vol. 221, 30 November 2023 (2023-11-30), NL, pages 105766, ISSN: 0166-3542, DOI: 10.1016/j.antiviral.2023.105766
- CHEN JUN ET AL: "Antiviral Activity and Safety of Darunavir/Cobicistat for the Treatment of COVID-19", OPEN FORUM INFECTIOUS DISEASES, vol. 7, no. 7, 21 June 2020 (2020-06-21), pages ofaa241, XP093074216, DOI: 10.1093/ofid/ofaa241
- CAO BIN ET AL: "A Trial of Lopinavir-Ritonavir in Adults Hospitalized with Severe Covid-19", THE NEW ENGLAND JOURNAL OF MEDICINE, vol. 382, no. 19, 7 May 2020 (2020-05-07), US, pages 1787 - 1799, XP055802868, ISSN: 0028-4793, Retrieved from the Internet <URL:https://www.ncbi.nlm.nih.gov/pmc/articles/PMC7121492/pdf/NEJMoa2001282.pdf> DOI: 10.1056/NEJMoa2001282
- BEIGEL JOHN H. ET AL: "Remdesivir for the Treatment of Covid-19 - Final Report", THE NEW ENGLAND JOURNAL OF MEDICINE, vol. 383, no. 19, 22 May 2020 (2020-05-22), US, pages 1813 - 1826, XP055775825, ISSN: 0028-4793, Retrieved from the Internet <URL:https://www.nejm.org/doi/pdf/10.1056/NEJMoa2007764?articleTools=true> DOI: 10.1056/NEJMoa2007764
- DE MEYER SANDRA ET AL: "Lack of antiviral activity of darunavir against SARS-CoV-2", INTERNATIONAL JOURNAL OF INFECTIOUS DISEASES, INTERNATIONAL SOCIETY FOR INFECTIOUS DISEASES, HAMILTON, CA, vol. 97, 29 May 2020 (2020-05-29), pages 7 - 10, XP086225609, ISSN: 1201-9712, [retrieved on 20200529], DOI: 10.1016/J.IJID.2020.05.085
- GUTIERREZ-VALENCIA ALICIA ET AL: "Darunavir/cobicistat showing similar effectiveness as darunavir/ritonavir monotherapy despite lower trough concentrations", JOURNAL OF THE INTERNATIONAL AIDS SOCIETY, vol. 21, no. 2, 12 February 2018 (2018-02-12), London, UK, pages e25072, XP055805129, ISSN: 1758-2652, DOI: 10.1002/jia2.25072
- ROBERTO PALUMBO ET AL: "Current treatment of COVID-19 in renal patients: hope or hype?", INTERNAL AND EMERGENCY MEDICINE, vol. 15, no. 8, 28 September 2020 (2020-09-28), pages 1389 - 1398, XP037350186, ISSN: 1828-0447, DOI: 10.1007/S11739-020-02510-0
- SHYTAJ IART LUCA ET AL: "Abstract", BIORXIV, 9 March 2021 (2021-03-09), XP055805295, Retrieved from the Internet <URL:https://www.biorxiv.org/content/10.1101/2021.03.09.434219v1.full.pdf> [retrieved on 20210518], DOI: 10.1101/2021.03.09.434219

## Description

The present invention relates to an oral dosage regimen for cobicistat for use in the prophylaxis and/or treatment of severe acute respiratory syndrome coronavirus type 2 (SARS-CoV-2) infection, severe acute respiratory syndrome coronavirus (SARS-CoV) infection and/or Middle East respiratory syndrome coronavirus (MERS-CoV) infection.

### BACKGROUND OF THE INVENTION

The announcement of the outbreak of Severe Acute Respiratory Syndrome Coronavirus type 2 (SARS-CoV-2) in December 2019 was followed by quick and pandemic spread of the infection, leading to a medical, economic and social crisis. One of the most challenging health emergencies of the past hundred years, the SARS-CoV-2 pandemic is highlighting the danger posed by RNA viruses, also in countries where they were absent or considered eradicated. The pathogenic effects of SARS CoV-2 can lead to the coronavirus disease 2019 (COVID-19), characterized by severe pneumonia with a high fatality rate, reaching a 40% among hospitalized old patients. Other coronaviruses associated with severe disease and high mortality are MERS-CoV, which can lead to the Middle East Respiratory Syndrome (MERS) and SARS-CoV, which bears a close genetic similarity with SARS-CoV-2 and was the causative agent of the Severe Acute Respiratory Syndrome (SARS).

SARS-CoV-2 belongs to the enveloped positive-sense RNA coronaviruses (Pal *et al.,* 2020), and its genome has a length of 29.9 kb with 12 functional open reading frames (ORFs), along with a set of 9 sub-genomic mRNAs which are carriers of a conserved leader sequence, 9 transcription-regulatory sequences, and 2 terminal untranslated regions (Fehr *et al.,* 2015). The genome encodes a total of 9,860 amino acids and, in particular, four main structural proteins: the spike (S)-glycoprotein, the small envelope/E glycoprotein, the membrane/M glycoprotein and the nucleocapsid/N protein (Pal et al., 2020, Jiang et al., 2020). Additionally, the SARS-CoV-2 genome encodes 16 non-structural proteins (NSPs), which encompass the two viral cysteine proteases, i.e. NSP3/papain-like protease and NSP5/3C-like protease (3CLpro, also known as main protease). Apartfrom the proteases, the viral NSPs encompass other key viral enzymes such as NSP12 /RNA-dependent RNA polymerase (RdRP) and NSP13/helicase, which are essential for the transcription and replication of the virus (Pal *et al.,* 2020).

The ongoing pandemic of SARS-CoV-2 poses the challenge of quick development of antiviral therapies. SARS-CoV-2 is an enveloped, positive sense, RNA virus of the *Coronaviridae* family, which includes other human-infecting pathogens such as SARS-CoV and MERS-CoV (V'kovski *et al.* 2020). Currently, there are no widely approved antivirals to treat infection with Coronaviruses. Substantial effort has been devoted to identifying inhibitors of SARS-CoV-2 replication through repurposing of compounds approved for treating other clinical indications. Repositioned drugs offer the advantage of a well-known safety profile and the possibility of faster clinical testing, which is essential during a sudden epidemic outbreak (Pushpakom *et al.* 2019). Large scale clinical trials have identified immune modulating agents (*e.g.* dexamethasone (Johnson and Vinetz 2020; RECOVERY Collaborative Group, Horby, Lim, et al. 2020)) as potential treatments for Coronavirus disease 2019 (COVID-19). However, direct acting antiviral agents have shown limited clinical benefits so far. In particular, a set of antiviral drugs initially identified as effective *in vitro* (remdesivir, chloroquine/hydroxychloroquine) has been unable to reproducibly decrease mortality in placebo-controlled trials (M. Wang *et al.* 2020; Beigel *et al.* 2020; Y. Wang *et al.* 2020; RECOVERY Collaborative Group, Horby, Mafham, et al. 2020).

Complete inhibition of SARS-CoV-2 replication will likely require combinations of antivirals, in line with previous evidence on other RNA viruses (Pawlotsky *et al.* 2015; Gulick and Flexner 2019). Candidate inhibitors have been proposed to target several critical steps of SARS-CoV-2 replication, including viral entry, polyprotein cleavage by viral proteases, transcription and viral RNA replication (Guy *et al.* 2020). SARS-CoV-2 entry is mediated by the spike glycoprotein (S-glycoprotein), which binds through its S1 subunit to the cellular receptor Angiotensinconverting enzyme 2 (ACE2). Upon binding, the viral entry requires a proteolytic activation of the S2 subunit leading to the fusion of the viral envelope with the host cell membrane (Hoffmann *et al.* 2020). The study of candidate inhibitors of SARS-CoV-2 entry has mainly focused on monoclonal antibodies and small molecules to target the association of the receptor binding domain (RBD) of the S-glycoprotein to ACE-2 (Xiu *et al.* 2020). Interestingly, the intensively studied antimalarials chloroquine and hydroxychloroquine have been suggested to impair SARS-CoV-2 entry *in vitro* by both decreasing the binding of the RBD to ACE2 and by decreasing endosomal acidification (Liu *et al.* 2020).

Upon viral membrane fusion, the viral RNA is released to the cytosol and translated into two large polyproteins that are cleaved into non-structural proteins (nsp) by two viral proteases, the main protease (3CLₚᵣₒ) and the papain-like protease (PLₚᵣₒ). A large body of work to identify antivirals against SARS-CoV-2 has focused on research on these viral proteases. Initial drug repurposing efforts focused on inhibitors of the HIV-1 protease, such as lopinavir and darunavir, alone or in combination with pharmacological boosters. These inhibitors, however, proved poorly effective in inhibiting 3CLₚᵣₒ activity *in vitro* (Mahdi *et al.* 2020) and did not offer reproducible clinical benefit (Cao *et al.* 2020; Chen *et al.* 2020; E. J. Kim *et al.* 2020). Larger drug screenings have so far relied on a combination of *in-silico* and *in vitro* tools (Jin *et al.* 2020). In particular, libraries of compounds have been screened through molecular docking and many candidate drugs have shown favorable binding properties to the SARS-CoV-2 proteases when analyzed by molecular dynamics (Razzaghi-Asl *et al.* 2020). Overall, however, repurposed inhibitors of SARS-CoV-2 proteases have generally shown half-maximal inhibitory concentration (IC50) values that were incompatible with dosages achievable *in vivo.*

The nsps generated by polyprotein cleavage by the viral proteases support the transcription and replication of the viral genome, which is catalyzed by the activity of the RNA-dependent RNA polymerase (RdRP). Owing to its crucial role and high evolutionary conservation, this viral enzyme represents a very attractive therapeutic target, which has so far been exploited by repurposing the anti-Ebola virus drug remdesivir (Mulangu *et al.* 2019; Beigel *et al.* 2020; Y. Wang *et al.* 2020). Other potential RdRP inhibitors, repurposed from treatment of HCV, HIV-1 and influenza virus have been proposed as well (Jácome *et al.* 2020; Chien *et al.* 2020; Jockusch *et al.* 2020). Among them, Favipiravir and Molnupiravir (MK-4482) have shown *in vivo* therapeutic potential by decreasing viral burden and transmission in hamster and ferret models of the infection, respectively (Cox, Wolf, and Plemper 2021; Kaptein *et al.* 2020). Viral transcripts generated by the RdRP are used for assembly of new virions by budding into the lumen of the ER-Golgi intermediate compartment (ERGIC) (Klein *et al.* 2020). The assembly is driven by the structural proteins M and E which are responsible for the incorporation of the N protein forming ribonucleoprotein complexes containing the viral genome. After the budding is completed, viruses are released from the cell either by exocytosis or through lysosomal organelle trafficking (V'kovski *et al.* 2020). So far, drug candidates proposed to target viral assembly/budding have not advanced beyond *in-silico* predictions (Gupta *et al.* 2020).

De Meyer S, et al. (De Meyer S, et al. Lack of antiviral activity of darunavir against SARS-CoV-2. Int J Infect Dis. 2020;97:7-10) discloses a dose of 150 mg cobicistat for use in the treatment of SARS-CoV-2 in combination with 800 mg darunavir. De Meyer S, et al. does not disclose that cobicistat taken orally alone at a dose of 300 to 1,000 mg daily can be for use in the prophylaxis and/or treatment of severe acute respiratory syndrome coronavirus type 2 (SARS-CoV-2) infection, severe acute respiratory syndrome coronavirus (SARS-CoV) infection and/or Middle East respiratory syndrome coronavirus (MERS-CoV) infection.

A major limitation hampering the development of combined antiviral strategies against SARS-CoV-2 is the lack of data on drug interactions. Initial guidelines have cautioned against the combined use of potentially effective compounds, such as remdesivir and chloroquine/hydroxychloroquine, on the basis of the possible interference of the latter with remdesivir metabolism through the efflux pump P-glycoprotein (P-gp) [(Gilead. Summary on compassionate use) (Leegwater *et al.* 2020; Arribas *et al.* 2020)]. On the other hand, extensive first pass metabolism by the liver is known to limit bioavailability of remdesivir forcing its intravenous administration, limiting both its scalability and, likely, antiviral efficacy (Siegel *et al.* 2017).

It is an objective of the present invention to provide means for antiviral therapies of SARS.

### SUMMARY OF THE INVENTION

The scope of the invention is defined by the appended set of claims.

According to the present invention this object is solved by providing cobicistat for use in the prophylaxis and/or treatment of severe acute respiratory syndrome coronavirus type 2 (SARS-CoV-2) infection, severe acute respiratory syndrome coronavirus (SARS-CoV) infection and/or Middle East respiratory syndrome coronavirus (MERS-CoV) infection,wherein cobicistat is administered orally at a daily dosage of 300 to 1,000 mg.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. Unless defined otherwise, all technical and scientific terms used herein have the same meanings as commonly understood by one of ordinary skill in the art.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 20" should be interpreted to include not only the explicitly recited values of 1 to 20, but also include individual values and sub-ranges within the indicated range. Thus, included in this numerical range are individual values such as 1, 2, 3, 4, 5 .... 17, 18, 19, 20 and sub-ranges such as from 2 to 10, 8 to 15. This same principle applies to ranges reciting only one numerical value, such as "higher than 150 mg per day". Furthermore, such an interpretation should apply regardless of the breadth of the range or the characteristics being described.

### Cobicistat for use in the prophylaxis and treatment of SARS-CoV-2

As outlined above, the present invention provides cobicistat for use in the prophylaxis and/or treatment of severe acute respiratory syndrome coronavirus type 2 (SARS-CoV-2) infection, severe acute respiratory syndrome coronavirus (SARS-CoV) infection and/or Middle East respiratory syndrome coronavirus (MERS-CoV) infection, wherein cobicistat is administered orally at a daily dosage of 300 to 1,000 mg.
*Name:* Cobicistat
*Other IDs:* GS 9350 / GS-9350 / GS9350
*DrugBank ID:* DB09065 (https://www.drugbank.ca/drugs/DB09065)
*ATC code:* V03AX03 (WHO) (https://www.whocc.no/atc_ddd_index/?code=V03AX03) *Chemical structure:* 1,3-thiazol-5-ylmethyl [(2R,5R)-5-{[(2S)-2-({[(2-isopropyl-1,3-thiazol-4-yl)methyl](methyl)carbamoyl}amino)-4-(morpholin-4-yl)butanoyl]amino}-1,6-diphenylhexan-2-yl]carbamate,
with a molecular formula of C₄₀H₅₃N₇O₅S₂ and a molecular weight of 776.0 g/mol (information retrieved from: National Center for Biotechnology Information. PubChem Database. Cobicistat, CID=25151504, https://pubchem.ncbi.nlm.nih.gov/compound/Cobicistat; accessed on June 16, 2020).

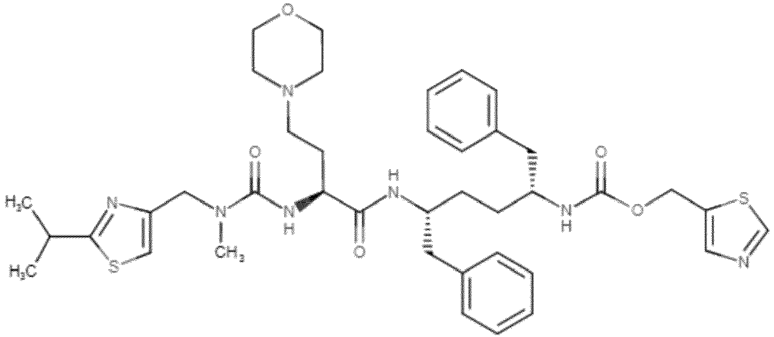

Cobicistat, marketed under the trade name of Tybost^{®}, is an approved therapy for treating HIV-1 infection. As of yet, cobicistat has not been used as a direct antretroviral, but rather exerts its effect as a pharmacokinetic enhancer (booster) for other antiretrovirals, such as the integrase inhibitor elvitegravir and some protease inhibitors (e.g. darunavir).

At a molecular level, cobicistat can selectively inhibit cytochrome P450 3A isoforms (CYP3A) and block P-glycoprotein efflux transporters, thus increasing the systemic exposure of coadministered agents, such as antiretroviral drugs, which are metabolized by CYP3A enzymes (von Hentig *et al.,* 2015).

Despite the existence of clinical guidelines and observations conducted by experts in the field, so far nobody hypothesized that cobicistat could be a direct-acting antiviral agent against SARS-CoV-2, as it was only described and utilized as a pharmacological booster of HIV-1 protease inhibitor. This lack of attention was likely due to the fact that it was known that Cobicistat is devoid of any direct antiviral activity against HIV-1, displaying an EC50 against the HIV-1 protease of more than 30 µM (https://www.selleckchem.com/products/cobicistat-gs-9350.html). Moreover, despite being approved by FDA since 2014, cobicistat was never tested during the MERS-CoV outbreak, for which, as in the case of SARS-CoV-2, there are no effective treatments.

Preferably, cobicistat is provided for use in the prophylaxis and/or treatment of Coronavirus disease 2019 (COVID-19).

Thereby, any stage of COVID-19 is comprised.

In a preferred embodiment, cobicistat is used in combination with one or more further drug.

Preferably, the one or more further drug is selected from
- an antiviral agent,
- an HIV protease inhibitor,
- plitidepsin,
- an anti-inflammatory glucocorticoid,
- a januskinase (JAK) inhibitor, and/or
- a palmitoyl protein thioesterase 1 *(*PPT1*)* inhibitor,
- a monoclonal antibody targeting viral replication or host inflammation.

Preferred antiviral agents are remdesivir, chloroquine or hydroxychloroquine, molnupiravir or favipiravir.

Preferred HIV protease inhibitors are tipranavir, nelfinavir, lopinavir and atazanavir.

Plitidepsin is a substrate of cytochrome P450-3As (CYP3A) and/or P-glycoprotein (P-gp).

Plitidepsin (aplidin) is metabolised through cytochrome P450-3A; the cellular target of cobicistat. It inhibits translation elongation factor eEF1A (White *et al.,* 2021).

Preferred anti-inflammatory glucocorticoids are dexamethasone, prednisone, methylprednisolone and hydrocortisone.

Preferred januskinase (JAK) inhibitors are baricitinib, ruxolitinib, and upadacitinib.

A preferred palmitoyl protein thioesterase 1 (PPT1) inhibitor is GNS561.

A preferred monoclonal antibody targeting host inflammation is tocilizumab.

In a preferred embodiment the further drug is remdesivir, i.a. a combination of cobicistat with remdesivir is preferred.

In one embodiment, the one or more further drug is remdesivir, tipranavir, chloroquine, hydroxychloroquine, molnupiravir, favipiravir, nelfinavir, lopinavir, atazanavir, plitidepsin, dexamethasone, baricitinib and/or GNS561.

In a preferred embodiment cobicistat is used in combination with remdesivir in further combination with one or more further drug, preferably with an HIV protease inhibitor, more preferably tipranavir, nelfinavir, lopinavir or atazanavir.

In one embodiment, cobicistat is used in combination with chloroquine in further combination with one or more further drug, preferably with an HIV protease inhibitor, more preferably tipranavir, nelfinavir, lopinavir or atazanavir.

### - Route of administration and therapeutically amount

A "therapeutically amount" or "therapeutically effective amount", both of which terms are used herein interchangeably, of cobicistat according to the present invention is the amount which results in the desired therapeutic result.

In HIV-1 treatment, Cobicistat is administered orally in combination with the HIV-1 protease inhibitors atazanavir (trade name of the combination: Evotaz^{®}) or darunavir (trade names of the combination: Prezcobix^{®} in the US and Rezolsta^{®} in the EU) or with a combination of several antiretrovirals (trade names Stribild^{®}, Genvoya^{®}, Symtuza^{®}). In all these fixed-dose combinations Cobicistat is administered in a tablet at 150 mg/day.

### Further description of preferred embodiments

Here, we demonstrate that the FDA-approved CYP3A inhibitor cobicistat, typically used as a booster of HIV-1 protease inhibitors (Sherman *et al.* 2015), can block SARS-CoV-2 replication *in vitro* in cell lines of lung and gut origin. While cobicistat was identified through *in-silico* screening of 3CLₚᵣₒ inhibitors, our data point towards an effect on the S-protein, which in the presence of cobicistat showed decreased ability to form syncytia in cells overexpressing the S-protein. The antiviral concentrations of cobicistat, while well tolerated *in vitro,* are clearly above those used for HIV-1 treatment, but compatible with plasma levels previously reached at higher doses in mice as well as in humans. In combination with remdesivir, cobicistat exhibits a synergistic effect in rescuing cell viability and abrogating viral replication in both cell lines and in a primary colon organoid. Overall, our data show that cobicistat has a dual activity both as antiviral drug and as pharmacoenhancer, thus potentially providing a basis for combined therapies aimed at complete suppression SARS-CoV-2 replication.

### - Abstract

Combinations of direct-acting antivirals are needed to minimize drug-resistance mutations and stably suppress replication of RNA viruses. Currently, there are limited therapeutic options against the Severe Acute Respiratory Syndrome Corona Virus 2 (SARS-CoV-2) and testing of a number of drug regimens has led to conflicting results. Here we show for the first time that cobicistat, which is an-FDA approved drug-booster that blocks the activity of the drug metabolizing proteins Cytochrome P450-3As (CYP3As) and P-glycoprotein (P-gp), can have antiviral activity and inhibit SARS-CoV-2 replication. This was unexpected as cobicistat was specifically developed to be "inert" against the HIV-1 protease and to exert solely a booster effect (Xu *et al.,* 2010). Our cell-to-cell membrane fusion assays indicated that the antiviral effect of cobicistat is exerted through inhibition of spike protein-mediated membrane fusion. Incubation with low micromolar concentrations of cobicistat decreased viral replication in three different cell lines including cells of lung and gut origin. These concentrations of cobicistat were previously deemed unnecessary as the inhibitory activity of the drug on CYP3A requires only low nanomolar concentrations (Xu *et al.,* 2010). Indeed, clinical trials testing drug regimens including cobicistat had only considered standard dosing of cobicistat and had not postulated any antiviral effect of this drug and were aimed solely at testing the antiviral activity of HIV-1 protease inhibitors (Chen et al. 2020). When cobicistat was used in combination with the putative CYP3A target and nucleoside analog remdesivir, a synergistic effect on the inhibition of viral replication was observed in cell lines and in a primary human colon organoid. The cobicistat/remdesivir combination was able to potently abate viral replication to levels comparable to mock-infected cells leading to an almost complete rescue of infected cell viability. These data highlight cobicistat as a therapeutic for treating SARS-CoV-2 infection and as a building block of combination therapies for COVID-19.

### - Results

### In-silico and in vitro analyses identify cobicistat as a candidate inhibitor of SARS-CoV-2 replication

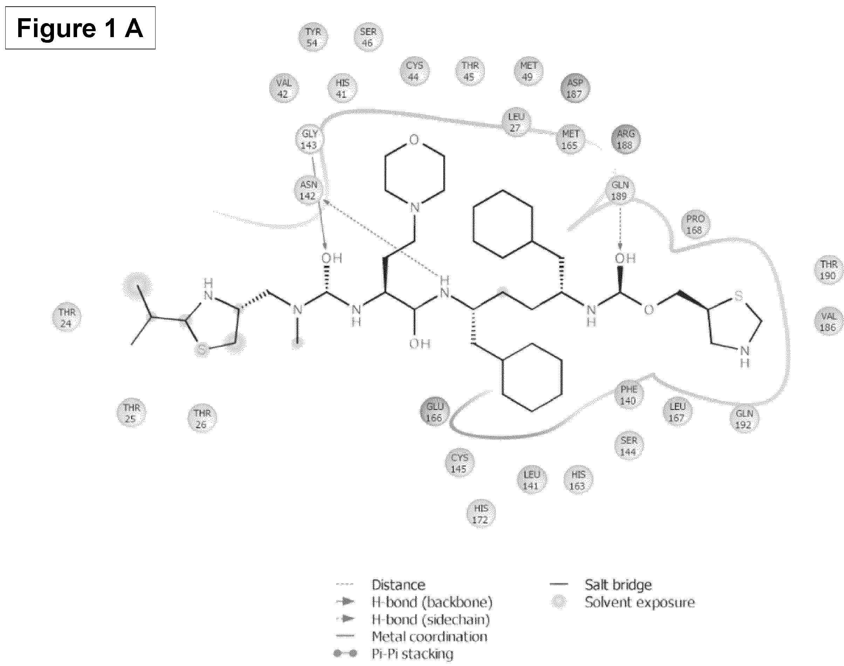

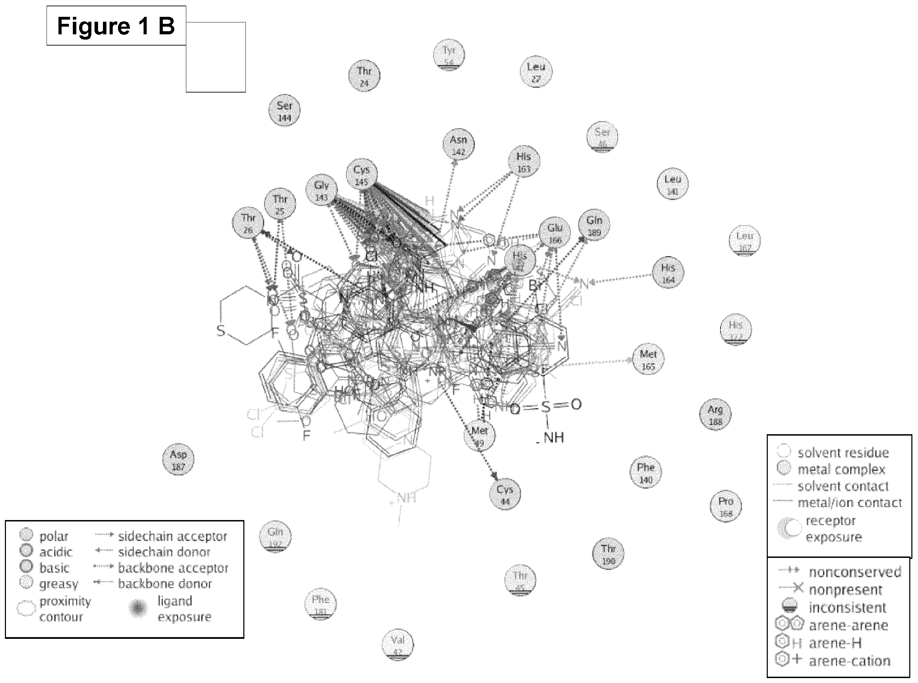

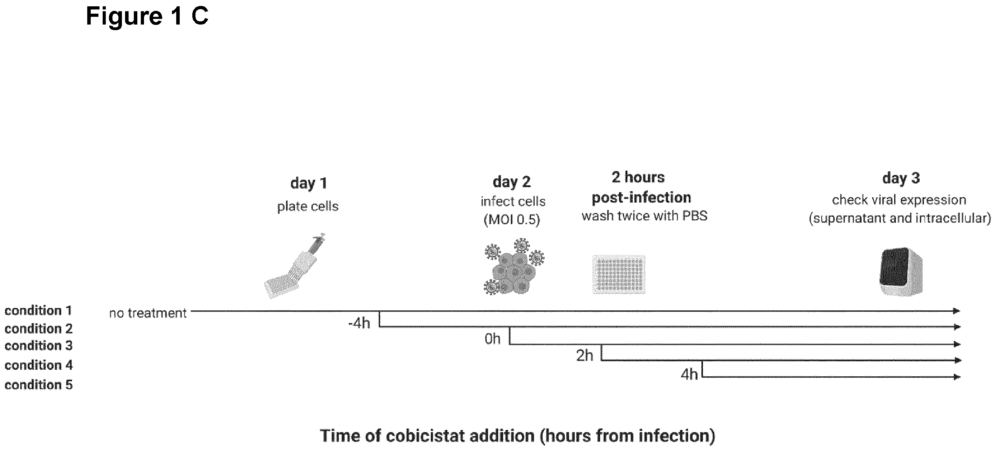

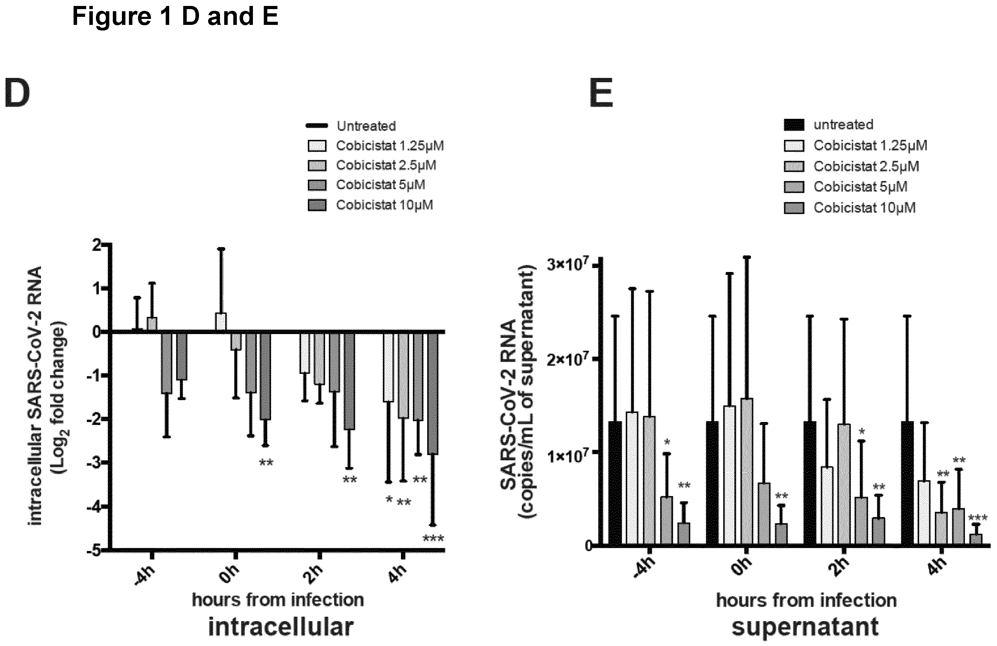

To identify potential inhibitors of SARS-CoV-2 replication we performed a structure-based virtual screening of the Drugbank library of compounds approved for clinical use. Candidate drugs were ranked based on their docking score to the substrate-binding site of 3CLₚᵣₒ, *i.e.* the site essential for the proteolytic function. Our results highlighted seventeen top candidate inhibitors, including compounds used to treat parasitic as well as viral infections. Among the latter, the HIV-1 protease inhibitor nelfinavir, which was one of the top scoring compounds in our analysis, was previously shown to decrease SARS-CoV and SARS-CoV-2 replication *in vitro* (Yamamoto *et al.* 2004, n.d.) (Table 1). Two additional drugs used for treatment of HIV-1 displayed top docking scores, *i.e.* the protease inhibitor tipranavir and, unexpectedly, the CYP3A inhibitor cobicistat, which was previously designed as a molecule devoid of antiviral activity (Xu *et al.,* 2010). The latter was a particularly interesting candidate, given its activity as a booster for HIV-1 protease inhibitors (Sherman *et al.* 2015), which renders it a promising candidate for combination therapies. Additional *in-silico* investigation of the binding poses and stability of cobicistat to the 3CLₚᵣₒ of SARS-CoV-2 corroborated a high predicted affinity for the target (Figure 1A and B). These *in-silico* results prompted us to test the effect of cobicistat on SARS-CoV-2 replication *in vitro.* For this purpose, we conducted a time course analysis of the effect of different concentrations of cobicistat on intracellular viral RNA replication and release of virus into the culture supernatant of Calu-3 cells (Figure 1C to E). Analysis of virus RNA amounts by qPCR showed a dose dependent inhibitory effect of low micromolar concentrations of cobicistat (Figure 1D and E). This effect was visible in both supernatants and cellular extracts, and was reproducible when samples were assayed with two different sets of primers [*i.e.* N1 and N2 primer sets recommended by the Center of Disease Control (Figure 1D and E with the N1 primer set; data for N2 primer set not shown)]. Of note, pre-incubation or treatment upon infection with cobicistat did not increase the antiviral effects as compared to adding the drug two or four hours post-infection, potentially suggesting an effect on late stages of the viral life cycle.

Taken together, these data show that cobicistat has a direct antiviral effect on SARS-CoV-2 replication *in vitro.*

**Table 1**

| **DRUGBANK _ID** | **Drug groups** | **Generic name** | **Main indication** | **Docking score** |
|---|---|---|---|---|
| DB01362 | approved | Iohexol | Contrast agent | -11.72 |
| DB09134 | approved | Ioversol | Contrast agent | -11.03 |
| DB 12407 | approved; investigational | Iobitridol | Contrast agent | -10.22 |
| DB12615 | approved; investigational | Plazomicin | Antibiotic for urinary tract infections | -9.43 |
| DB00932 | approved; investigational | Tipranavir | HIV protease inhibitor | -8.06 |
| DB00220 | approved | Nelfinavir | HIV protease inhibitor | -7.91 |
| DB08909 | approved | Glycerol phenylbutyrate | Nitrogen-binding agent for management of urea cycle disorders | -7.86 |
| DB00905 | approved; investigational | Bimatoprost | Analog of prostaglandin F2α for treatment of glaucoma | -7.67 |
| DB08889 | approved; investigational | Carfilzomib | Proteasome Inhibitor (anticancer) | -7.54 |
| **DB09065** | **approved** | **Cobicistat** | **CYP3A inhibitor for boosting HIV-1 protease inhibitors** | **-7.12** |
| DB04868 | approved; investigational | Nilotinib | Tyrosine kinase inhibitor for treatment of chronic myelogenous leukemia | -7.05 |
| DB01288 | approved; investigational | Fenoterol | Beta adrenergic agonist for asthma treatment | -7.05 |
| DB00482 | approved; investigational | Celecoxib | Nonsteroidal anti-inflammatory drug | -6.80 |
| DB13931 | approved | Netarsudil | Rho kinase inhibitor for treatment of glaucoma | -6.75 |
| DB11611 | approved | Lifitegrast | Anti-Inflammatory for treatment of keratoconjunctivitis sicca | -6.45 |
| DB11979 | approved; investigational | Elagolix | gonadotropin-releasing hormone antagonist for treatment of endometriosis pain | -5.72 |
| DB01116 | approved; investigational | Trimethaphan | nicotinic antagonist used to counteract hypertension | -5.70 |

### The antiviral concentration range of cobicistat is well tolerated in vitro, but above plasma levels equivalents achievable through standard dosing of the drug

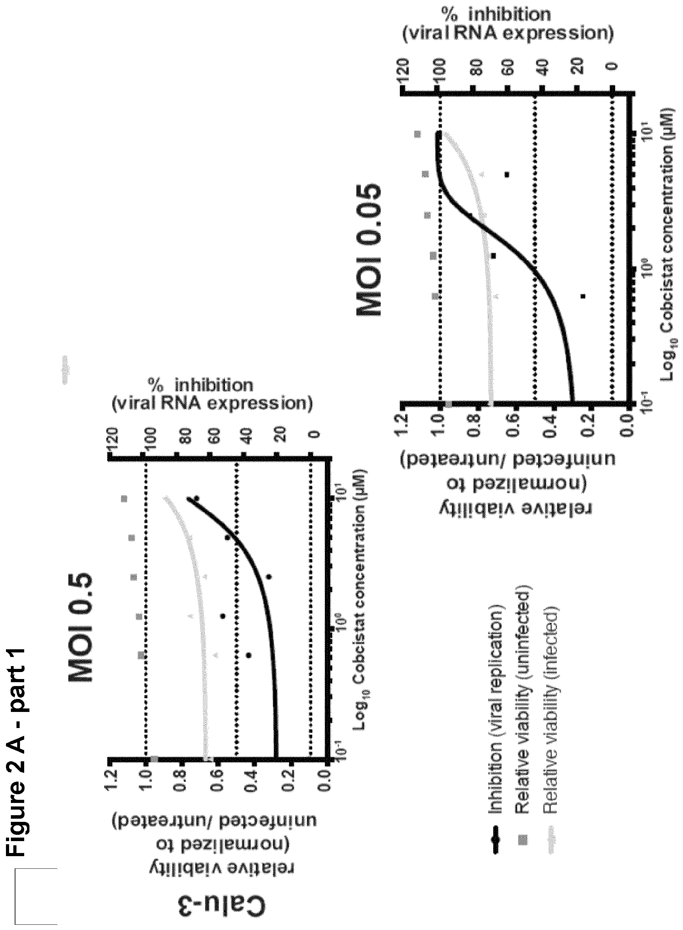

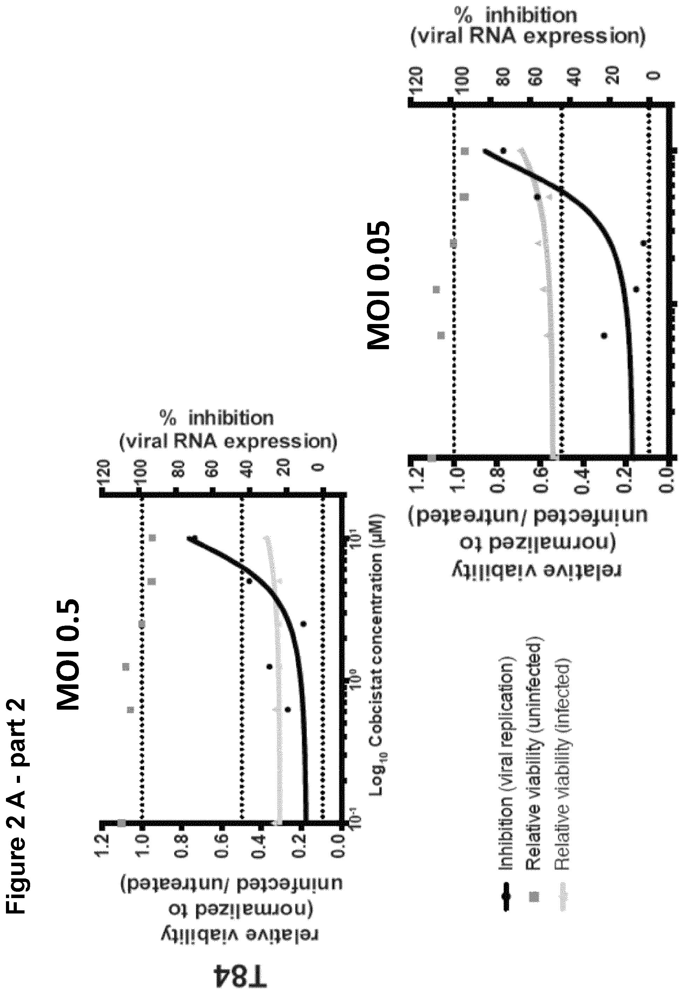

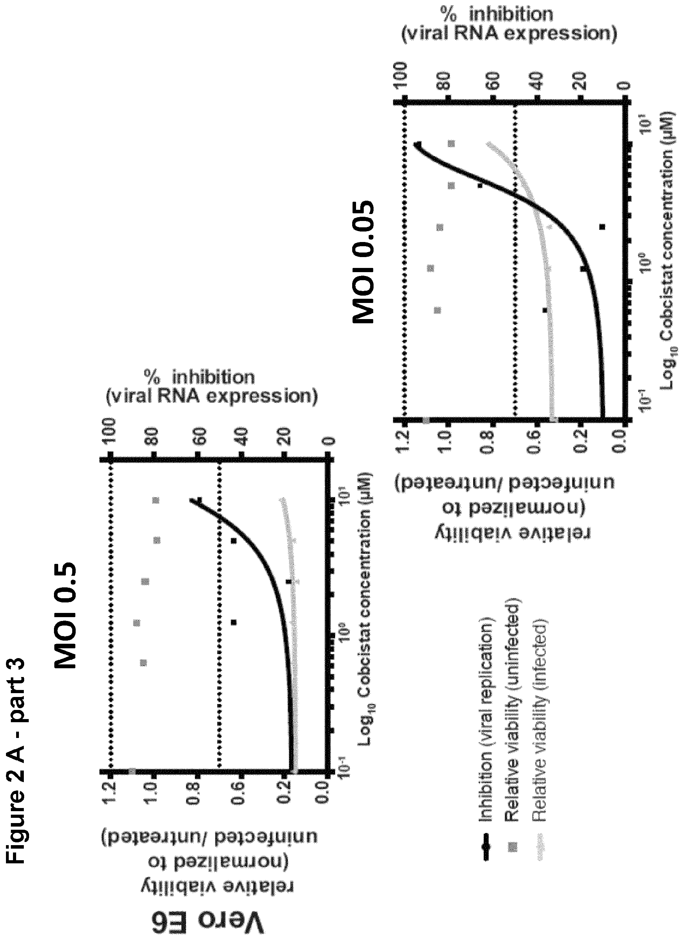

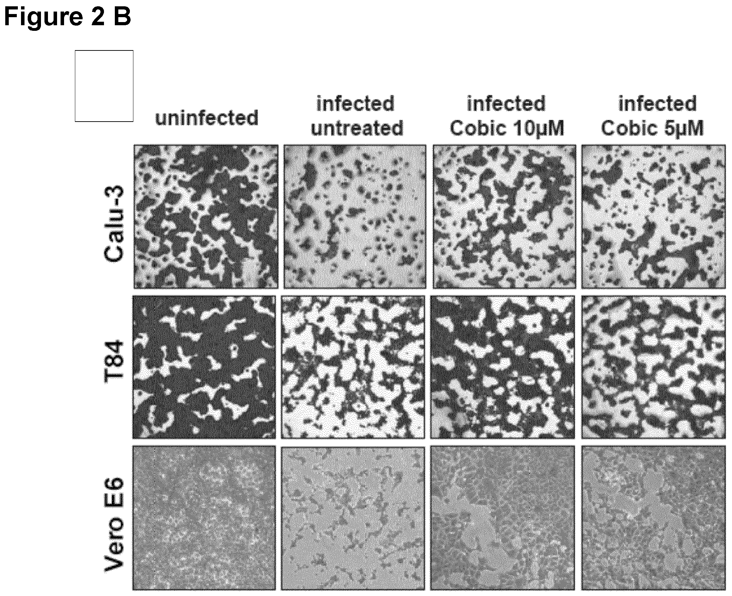

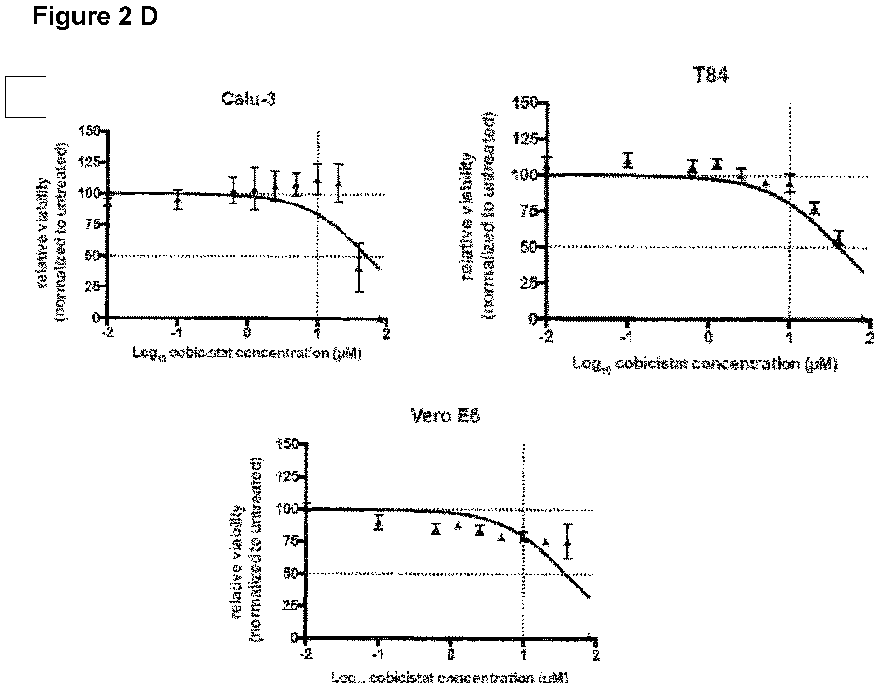

We next analyzed more thoroughly the antiviral effects of cobicistat using three cell lines of different origin, *i.e.* Calu-3 cells (human lung), Vero E6 cells (african green monkey kidney) and T84 cells (human gut), to reflect various known or putative tissue compartments of SARS-CoV-2 replication. Each cell line was infected using two different multiplicities of infection [(MOI) 0.05 and 0.5] and left untreated or treated with various concentrations of cobicistat 2h post-infection. In all cell lines, cobicistat showed a dose dependent effect in decreasing viral RNA release in supernatant (Figure 2A). In line with this, the higher concentrations of cobicistat tested (5-10µM) were able to partially rescue viability of infected cells, as shown by both MTT and crystal violet assay (Figure 2A and B), while being well tolerated by uninfected cells (Figure 2A and D). Overall, the range of IC50 concentrations of cobicistat (0.58-8.76µM) was dependent on the MOI of the infection and on the cell type, but always far below the half cytotoxic concentration (CC50) range of the drug on the same cell types (38.66-53µM). We then compared our *in vitro* results with previously known pharmacokinetic properties of cobicistat in humans and mice, namely the peak plasma levels detectable in mice (Pharmacology Review of Cobicistat - Application number: 203- 094) and in humans (Mathias *et al.* 2010; Kakuda *et al.* 2014).

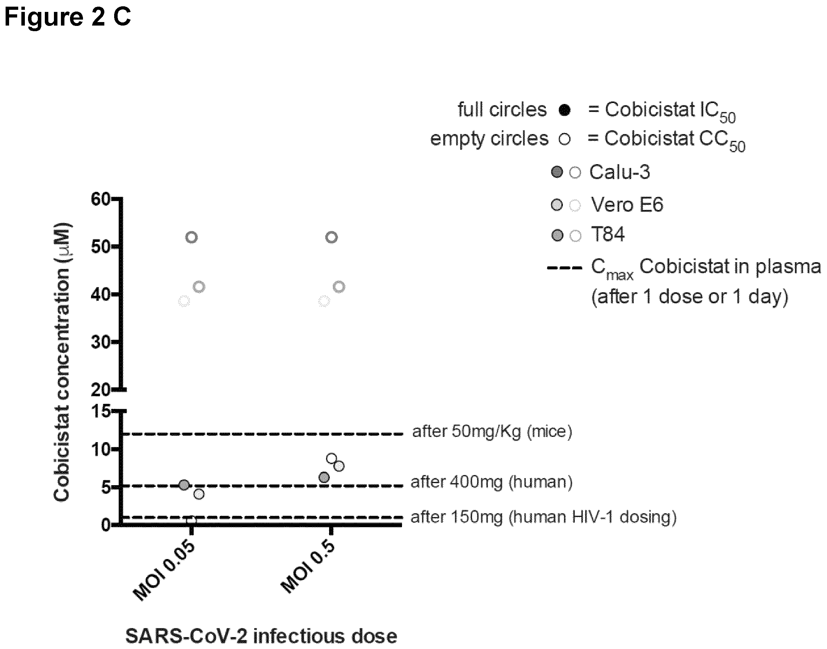

Interestingly, maximum plasma concentrations achievable through standard dosing of cobicistat (150mg/day as a booster for HIV-1 protease inhibitors) (Deeks 2014) were well below (≈1µM) most IC50 values obtained in our experiments (Figure 2C). In line with this, clinical testing of the HIV-1 protease darunavir, boosted by standard concentrations of cobicistat, did not yield clinical benefit to SARS-CoV-2 infected patients (Chen *et al.* 2020). On the other hand, plasma levels achievable through a higher dosage of cobicistat, that was tested in tolerability studies of this drug (400mg/day) (Mathias *et al.* 2010), were above IC50 values calculated when cells were infected using a 0.05 MOI (Figure 2C). Moreover, plasma levels achievable in mice through a higher cobicistat dosage shown to be safe in this animal model (50mg/Kg) were clearly above all IC50 values calculated in our experiments, while remaining below the CC50 concentrations (Figure 2C).

Overall, our data show that non-toxic concentrations of cobicistat can consistently decrease SARS-CoV-2 replication in various cellular infection models. Moreover, these data prove that plasma concentrations obtained through standard HIV-1 dosing of cobicistat are below those required to highlight the antiviral effect of cobicistat.

### Cobicistat decreases S-glycoprotein content and syncytia formation in vitro

To characterize the mechanism of the antiviral effects of cobicistat, we analyzed the catalytic activity of 3CLₚᵣₒ using a previously described FRET assay (Zhang *et al.* 2020). Apart from cobicistat, compounds tested included HIV-1 protease inhibitors highlighted by our molecular docking [nelfinavir, tipranavir] or previously administered in clinical trials as SARS-CoV-2 therapeutics [darunavir (Chen *et al.* 2020), lopinavir (Cao *et al.* 2020)], as well as two positive controls known to inhibit 3CLₚᵣₒ activity [MG132 and GC376 (Ma *et al.* 2020)].

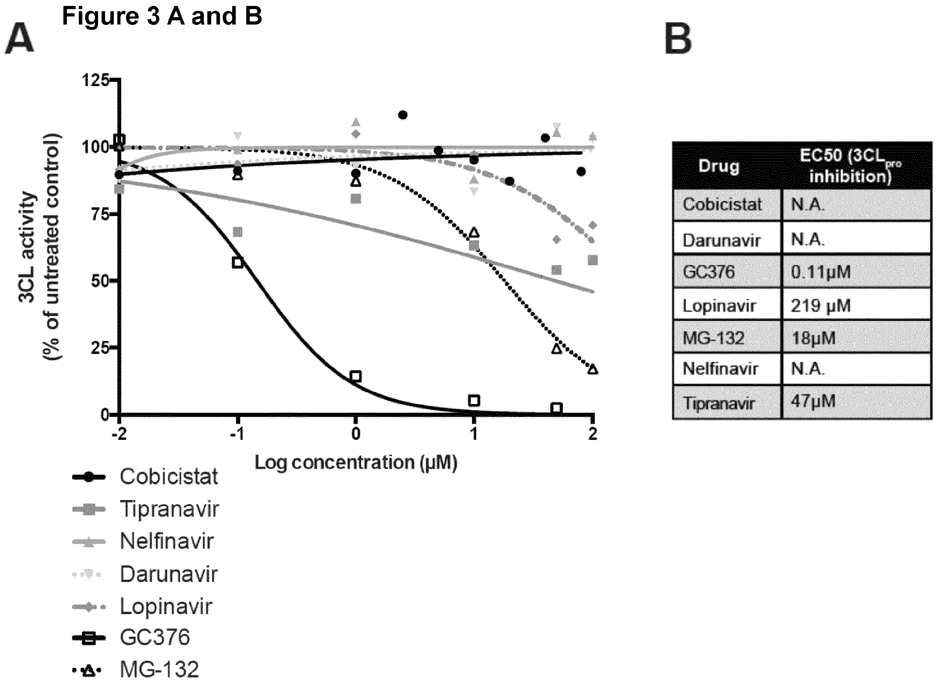

While treatment with the known inhibitors of 3CLₚᵣₒ, such as GC376 and MG-132, potently reduced the catalytic activity of the enzyme, cobicistat was surprisingly inactive (Figure 3A and B). Among the top scoring compounds in our docking analysis (Table 1), only tipranavir proved able to partially inhibit 3CLₚᵣₒ activity, although at relatively high concentrations (EC50 47µM; Figure 3A,B).

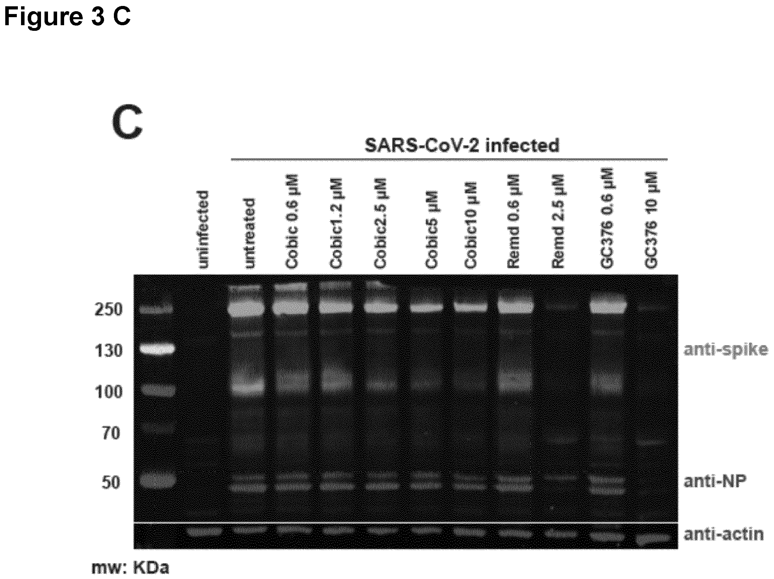

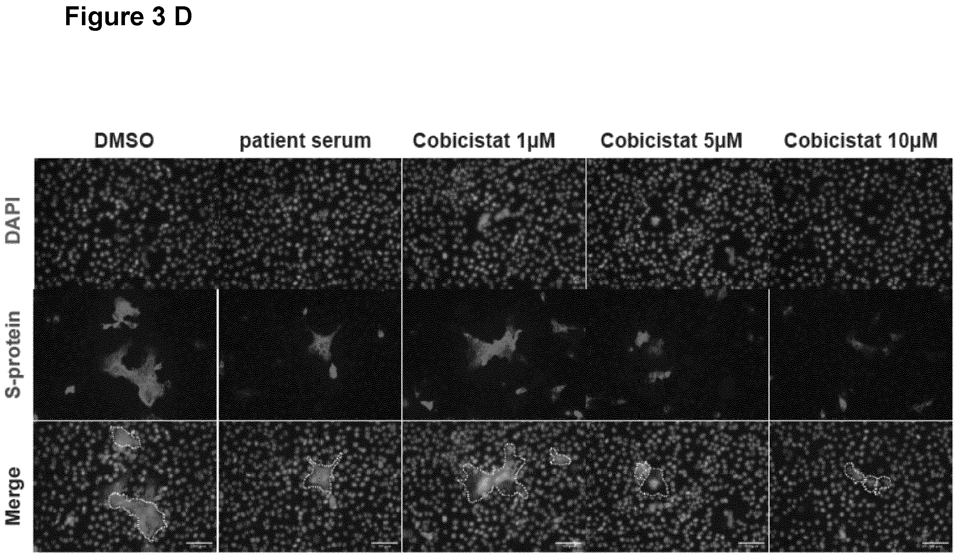

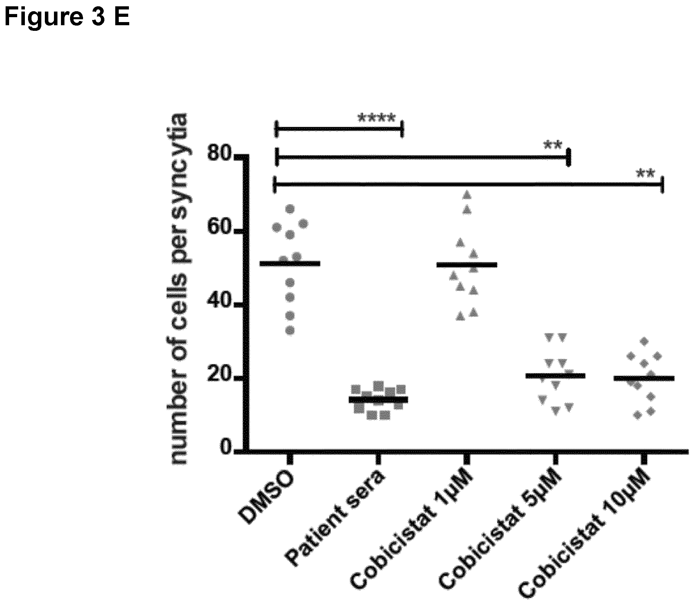

In light of the lack of effect of cobicistat on 3CLₚᵣₒ, we proceeded to analyze the possible impact of cobicistat on other key viral proteins. To reduce the bias of the analysis, while retaining a representative model of the infection, we performed western blot analysis of Vero E6 cell lysates using previously validated patient sera to detect viral proteins (Pape *et al.* 2020). The results showed the reduction of a high molecular weight band (≈250 kDa) when infected cells were incubated with low micromolar concentrations of cobicistat (data not shown). Based on the known molecular weights of SARS-CoV-2 proteins, we postulated that the patterns detected with patient sera corresponded to dimers/trimers of the S-protein (Ou *et al.* 2020; Algaissi *et al.* 2020) and to the nucleoprotein (N-protein) (Algaissi *et al.* 2020) of the virus. To confirm this hypothesis, we performed western blot analysis using monoclonal antibodies against the S and N proteins (Figure 3C). The results confirmed the observation that S-protein levels are decreased by cobicistat (Figure 3C). Moreover, the data indicated a high inhibition at the level of the S2 subunit (≈100 KDa) of the S-protein, *i.e.* the subunit responsible for the fusion to the host cell and subsequent viral entry (Figure 3C). To isolate the possible effect of cobicistat on S-protein-mediated fusion, we used a cellular assay measuring syncytia formation in Vero E6 cells transfected with the S-protein. The results showed decreased syncytia formation when cells were incubated with cobicistat or when sera from SARS-CoV-2 patients were used as controls to block S-protein fusion (Figure 3D and E). Of note, both western blot analysis and the syncytia assay indicated an effect of cobicistat in the 5-10µM range, which corresponds to the range of most IC50 values calculated on the basis of viral RNA levels in supernatants (Figure 2A, C).

Overall, these data show that the antiviral effect of cobicistat is not mediated by inhibition of 3CLₚᵣₒ activity, but is rather exerted, at least partially, through impairment of S-protein-mediated fusion.

### Cobicistat potently enhances the antiviral effect of remdesivir in cell lines and a primary colon organoid

We then tested the potential of cobicistat to exert a double activity as direct inhibitor of SARS-CoV-2 replication and as pharmacoenhancer of other antivirals. To this aim, we evaluated remdesivir as a candidate compound to synergize with cobicistat. The choice of remdesivir was motivated by its known activity as an inhibitor of SARS-CoV-2 RdRP, as well as by its postulated susceptibility to extensive first pass liver metabolism, potentially mediated by the cellular targets of cobicistat CYP3A and P-gp [E.M.A., Human Medicines Division. Summary on compassionate use Remdesivir Gilead (Siegel *et al.* 2017)]. We thus examined the *in silico* predicted affinity of remdesivir for the main members of the CYP3A family (CYP3A4 and 5), as well as for P-gp. The SwissADME server (Daina, Michielin, and Zoete 2017) predicted remdesivir to be both a CYP3A4 and P-gp substrate by using machine learning models with 79% and 88% accuracy, respectively. Similarly, the pkCSM (Pires, Blundell, and Ascher 2015) and CYPreact (Tian *et al.* 2018) servers also predicted remdesivir to be both a P-gp and CYP3A4 substrate, but not an inhibitor. Finally, remdesivir displayed high docking scores to the active sites of CYP3A4, CYP3A5 and P-gp (data not shown), which were comparable to those of ritonavir and cobicistat, *i.e.* known inhibitors with well characterized binding (data not shown).

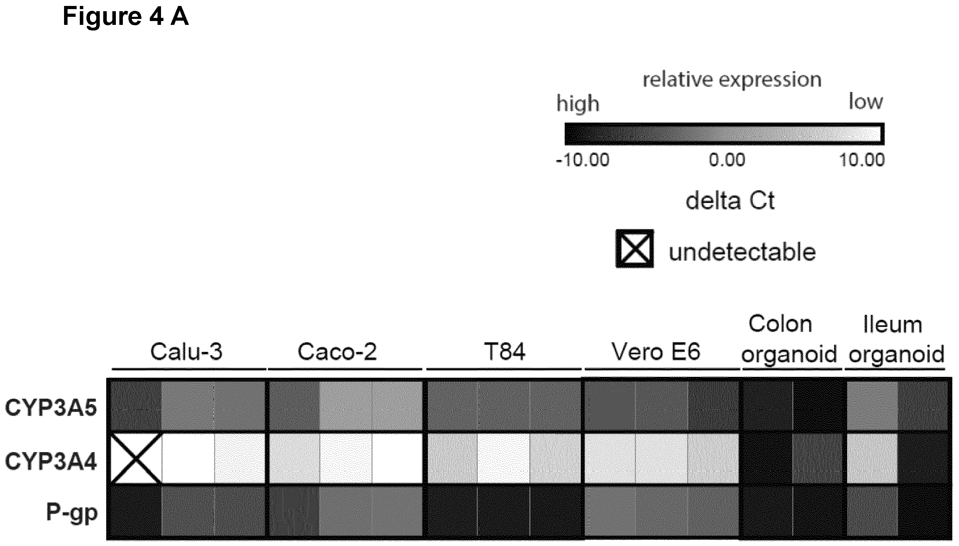

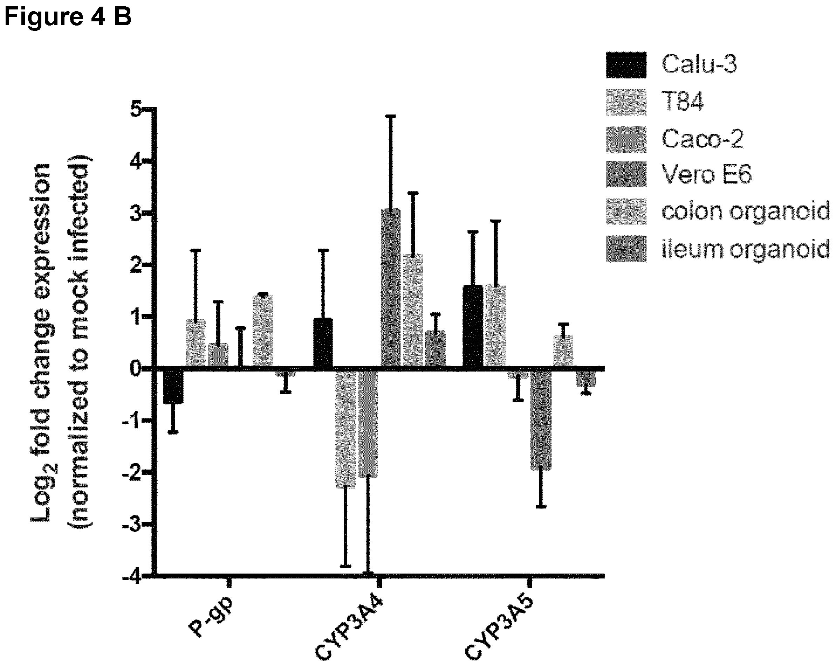

To identify the most suitable *in vitro* model for testing the combination of remdesivir and cobicistat, we first examined the relative expression levels of CYP3A4, CYP3A5 and P-gp in different human tissues and cell lines susceptible to SARS-CoV-2 infection (Figure 4). Both transcriptomic analysis (data not shown) and qPCR analysis highlighted liver, gut and kidney as major compartments of CYP3A4/5 and P-gp expression (Figure 4A), as previously described (von Richter *et al.* 2004; Wessler *et al.* 2013). On the other hand, primary lung tissues were characterized by lower CYP3A4/5 and P-gp expression, while the cell line Calu-3 showed intermediate characteristics, with low CYP3A4/5 and high P-gp expression, in line with upregulation of the latter marker in cancer cells (Bradley and Ling 1994). Of note, SARS-CoV-2 infection was associated with altered expression of these genes. Overall, infected cells and primary gut organoids showed a trend towards upregulation of P-gp, CYP3A4 and CYP3A5 expression (Figure 4B). Of note, cell lines of gut origin and Vero E6 cells displayed a peculiar trend showing opposite expression patterns of CYP3A4 and CYP3A5 upon infection (Figure 4B). Given their divergent response to the infection, we decided to use both Vero E6 and T84 cells as models for testing cobicistat and remdesivir, to obtain data on the efficacy of the drug combination and on its possible reliance on increased expression of either CYP3A4 or CYP3A5.

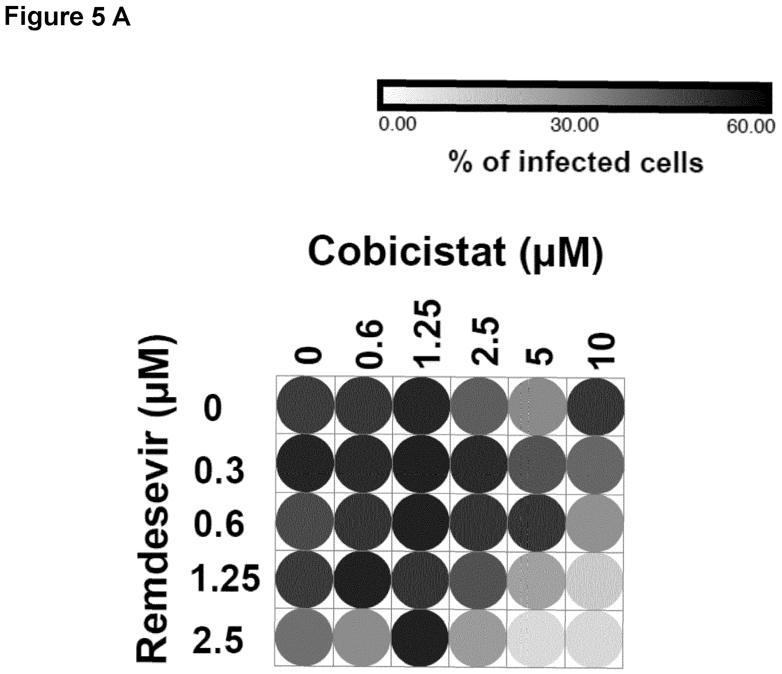

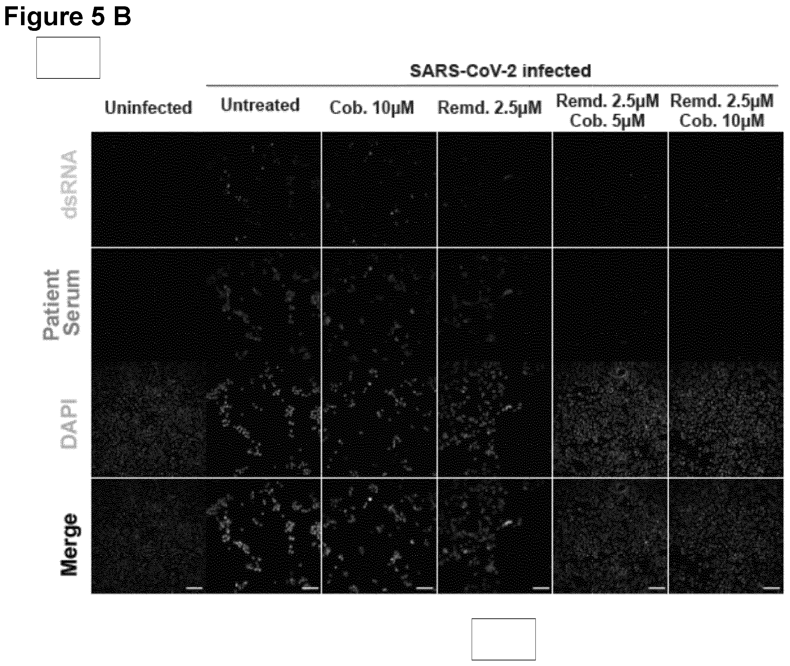

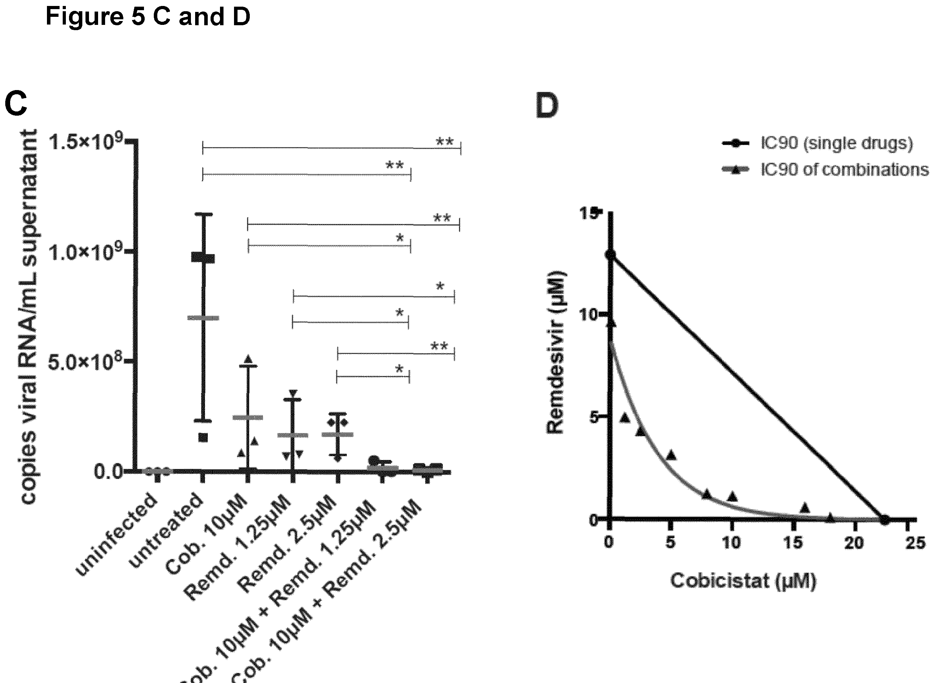

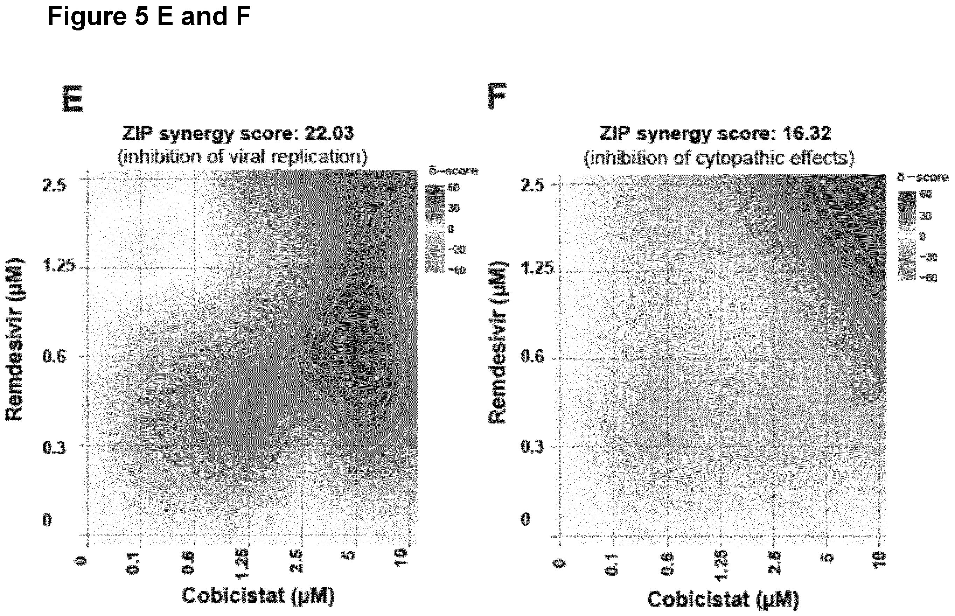

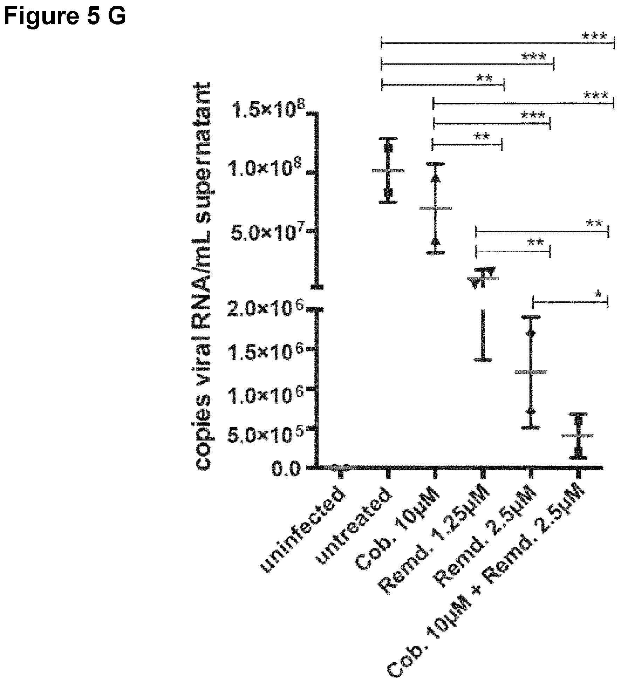

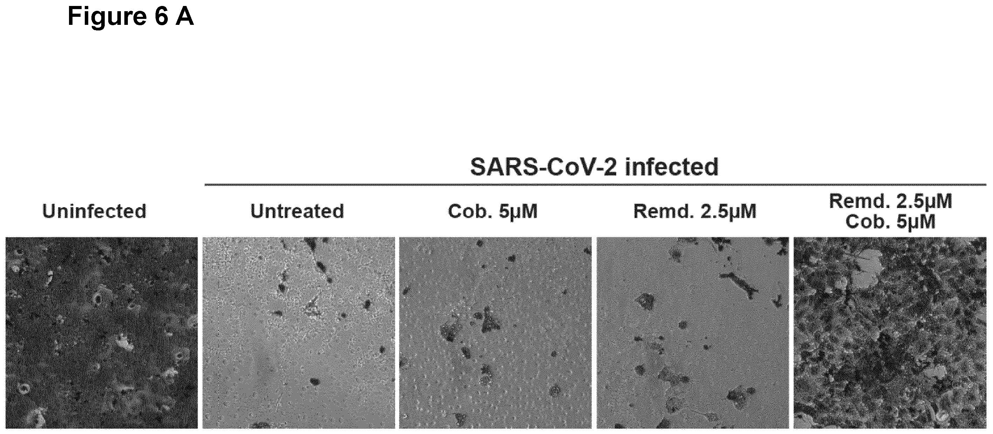

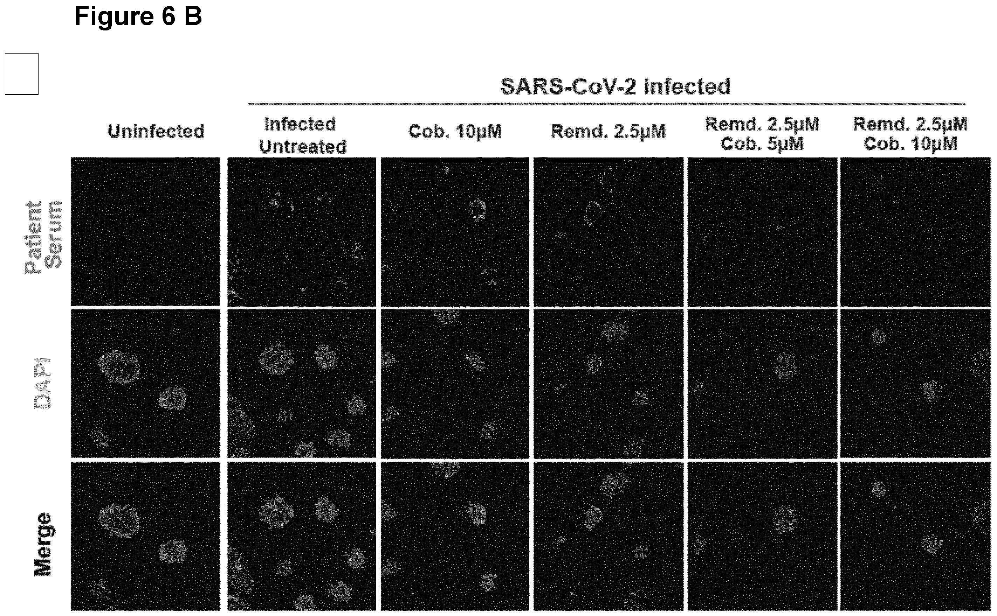

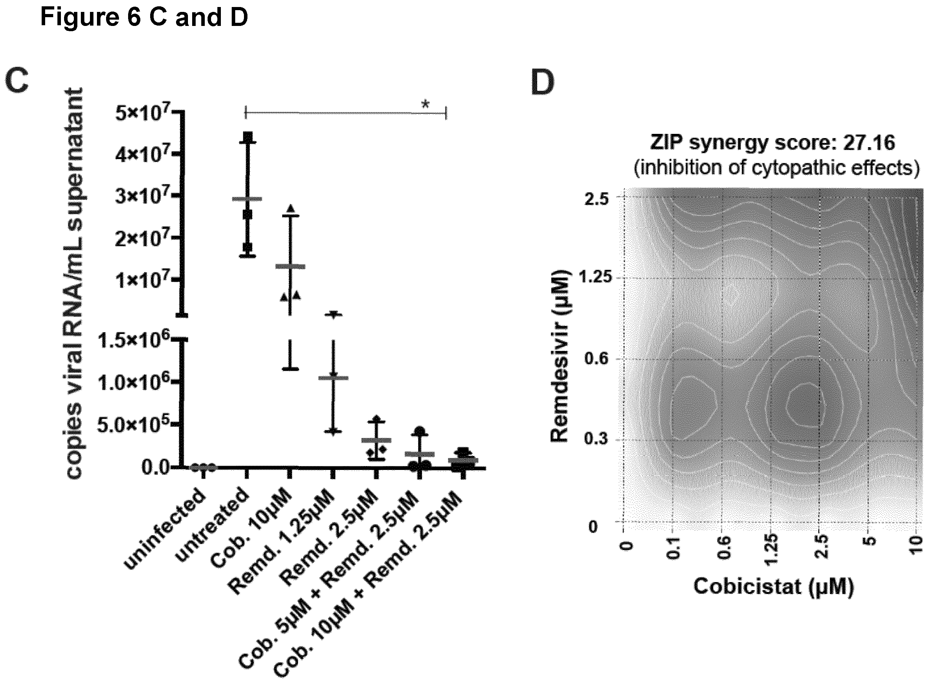

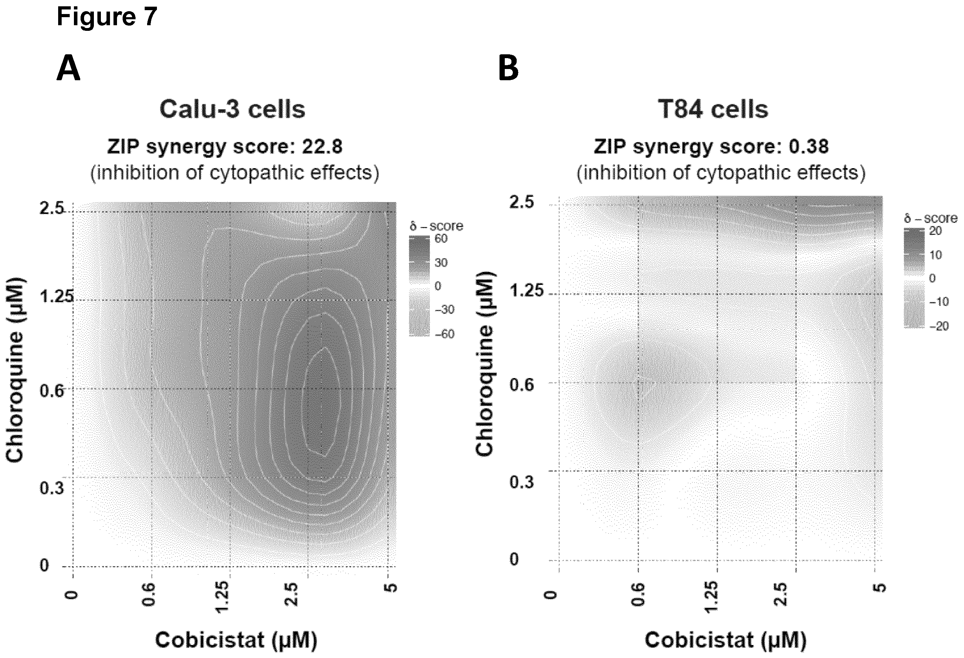

While treatment with remdesivir-only displayed antiviral activity at previously described levels (data not shown), the combined use of cobicistat and remdesivir was able to significantly enhance the effect of each drug alone, in both cell lines (Figure 5 A-G, and Figure 6A-D). Surprisingly, this synergistic effect was most visible when cobicistat was administered at low micromolar concentrations, thus proving that the synergism is not merely driven by a booster effect of cobicistat, but also by its direct antiviral activity which had never before been postulated by people skilled in the art. In particular, the drug combination was able to almost completely abrogate viral infection/replication, as measured by IF (Figure 5 A, B; Figure 6B), and qPCR (Figure 5C-E; Figure 6C), analysis. In line with this potent antiviral activity, the cobicistat/remdesivir combination also displayed a synergistic effect in inhibiting the cytopathic effects of SARS-CoV-2, thus restoring viability of infected cells to levels comparable to mock-infected controls (Figure 5F; Figure 6A,B,D). We then tested the effect of the drug combination on a primary human colon organoid (Figure 5G), which is susceptible to SARS-CoV-2 infection, as previously described (Stanifer *et al.* 2020). Also in this case, the addition of cobicistat enhanced the antiviral effect of remdesivir. Finally, we tested the ability of a combination of cobicistat and chloroquine in rescuing cytopathic effects of SARS-CoV-2 infection in two cell lines (Figure 7A and B). While the effects of this combination were lower than those observed when treating with cobicistat and remdesivir, the use of chloroquine with cobicistat was synergistic in one of the two cell lines considered (*i.e*. Calu-3 cells) (Figure 7A).

Overall, our data prove that the combination of cobicistat and remdesivir can suppress viral replication in different cellular models of SARS-CoV-2 infection and show that cobicistat can exert a double activity as direct antiviral agent and as pharmacoenhancer.

### - Discussion

The data herein presented demonstrate the antiviral activity of the FDA-approved drug cobicistat and support its role for combined antiviral therapies against SARS-CoV-2. The use of drug combinations targeting different steps of the viral life cycle is a well-established paradigm for treating RNA-virus infections (Bartlett *et al.* 2001; Naggie and Muir 2017). Translating this concept to SARS-CoV-2 drug development has, however, proven challenging due to the paucity of effective drug candidates available. In particular, compounds showing promise in initial studies, have failed to reproducibly decrease the mortality and morbidity of the infection (M. Wang *et al.* 2020; Beigel *et al.* 2020; Y. Wang *et al.* 2020; RECOVERY Collaborative Group, Horby, Mafham, *et al.* 2020). Similarly disappointing results were observed in the early stages of HIV-1 drug discovery, and might be partially explained by the inability of candidate antivirals to reach *in vivo* concentrations sufficient to completely block viral replication. The use of pharmacoenhancers such as cobicistat (Sherman *et al.* 2015) could help to overcome these limitations.

While the present study exclusively focused on the combination of cobicistat and remdesivir, more than 30% of all drugs are metabolized by the main cellular targets of cobicistat *(i.e.* CYP3A4/5) (van Waterschoot *et al.* 2010). For example, the recently described SARS-CoV-2 inhibitor plitidepsin (White *et al.* 2021) is mainly metabolized by CYP3A4 *in vitro* (Brandon *et al.* 2007). Therefore, it is conceivable that a synergistic effect similar to that described for remdesivir can be obtained by coupling cobicistat to other antiviral agents. In particular, other compounds tested in clinical trials of SARS-CoV-2 patients, such as chloroquine/hydroxychloroquine (K.-A. Kim *et al.* 2003) and lopinavir (van Waterschoot *et al.* 2010), are well known substrates of CYP3A. The booster effect of cobicistat would be further complemented by the own antiviral activity of this drug, which was proven herein *in vitro* on several models of SARS-CoV-2 infection. In line with this, we observed the strongest synergistic effect with remdesivir, when cobicistat was used at concentrations above its IC50 levels, suggesting that the hitherto unknown antiviral effects of cobicistat contribute to the observed synergism. Of note, the concentration range in which cobicistat could inhibit SARS-CoV-2 replication was higher than that achievable through standard dosages *(i.e.* 150mg/day) approved for treatment of HIV-1 infection (Deeks 2014). Therefore, the antiviral effect of cobicistat requires administration of the drug at higher dosages (e.g. 400mg/day) which result in plasma levels compatible with the antiviral concentrations described in our study (Mathias *et al.* 2010). These observations can also explain the lack of success of an early trial testing the HIV-1 protease inhibitor darunavir, boosted by a standard cobicistat dose (Chen *et al.* 2020). It is important to note that the authors of said study never considered the addition of cobicistat to have any antiviral potential and concluded from the study the lack of antiviral activity of darunavir, without hypothesizing the possibility to increase the dosage of cobicistat.

Another possible limitation of candidate antivirals for SARS-CoV-2 treatment is the inability to reach specific tissue reservoirs of the infection. Remdesivir is case in point, due to its quick metabolization and poor intestinal absorption (Hu *et al.* 2020). Of note, previous experience with HIV-1 protease inhibitors suggests that cobicistat might overcome this limitation (Lepist *et al.* 2012), in line with the synergistic effect that we observed when treating primary colon organoid and T84 colon adenocarcinoma cells with the combination of cobicistat and remdesivir. Intriguingly, the tissue penetration and activity of cobicistat in the main sites of CYP3A expression (*i.e.* gut and liver) can be relevant also for the route of administration of remdesivir. Currently, remdesivir requires intravenous administration due to its extensive first pass metabolism (Jorgensen, Kebriaei, and Dresser 2020), but its coupling with cobicistat can improve its absorption, perhaps allowing oral formulation of the drug. Increasing the scalability of remdesivir might *per se* improve its therapeutic potential, as an early treatment of the infection might prevent hospitalization and development of severe COVID-19, a stage where the efficacy of remdesivir could not be firmly established (Y. Wang *et al.* 2020).

Overall, our study introduces cobicistat as an agent for inhibiting SARS-CoV-2 replication and for combination therapies aimed at blocking or reversing the onset of COVID-19.

The following examples and drawings illustrate the present invention without, however, limiting the same thereto. The invention is defined solely by the claims.

### BRIEF DESCRIPTION OF THE DRAWINGS

**Figure 1** *Cobicistat is an inhibitor of SARS-CoV-2 replication.*
   A-C) *In-silico* docking (A,B) and molecular dynamics (C-E) analysis of the putative mode and energy of binding of cobicistat to SARS-CoV-2 3 CLₚᵣₒ.
   A) Docking pose showing the ligand interaction of cobicistat to the active site of 3CLₚᵣₒ and the formation of hydrogen bonds to ASN142, GLY143 and GLN189 of 3CLₚᵣₒ.
   B) Overlay of crystal structures of SARS-Cov-2 3CLₚᵣₒ showing the amino acids important for the binding of cobicistat to the active site of the enzyme. Residues of the catalytic dyad (Cys145 and His41) of 3CLₚᵣₒ were among the highest contributors to non covalent binding to cobicistat. The source and list of structures used are detailed in Example 1.
   C) Schematic representation of time course experiments evaluating *in vitro* inhibition of SARS-CoV-2 replication by cobicistat.
   D,E) Effect of various concentrations of cobicistat, added according to the scheme of (D) on intracellular and supernatant SARS-CoV-2 RNA content in Calu-3 cells. Viral RNA content was measured by qPCR using the 2019-nCoV_N1 primer set (Center of Disease Control). Fold change values in intracellular RNA (D) were calculated by the delta-delta CT method, using the Tata-binding protein (*TBP*) gene as housekeeper control. Expression levels in supernatant
   (E) were quantified using an *in vitro* transcribed standard curve generated as described in Example 1. Data are expressed as mean with SD and were analyzed by two-way ANOVA followed by Dunnet's post-test (N = 3 independent experiments). * P < 0.05; ** P < 0.01; *** P < 0.001.
**Figure 2****.** *Cobicistat decreases replication of SARS-Co V-2 and rescues viability of infected cells in multiple in vitro models.*
   A,B) Effect of serial dilutions of cobicistat on SARS-CoV-2 RNA concentration in supernatants (A) and on the viability of infected and uninfected cell lines of lung (Calu-3), gut (T84) and kidney (Vero E6) origin (A,B). Cells were infected with SARS-CoV-2 at two different MOIs (0.05 and 0.5) and left untreated or treated with cobicistat two hours post-infection. Forty-eight hours post-infection supernatants were collected and viral RNA was assayed by qPCR while cellular viability was measured by MTT assay (A) or by crystal violet staining (B). Inhibition of viral replication was calculated as described in Example 1 while viability data were normalized to the uninfected or to the untreated control. Half maximal inhibitory (IC50) concentration values were calculated by nonlinear regression. Each point in panel A represents a mean of 3 independent experiments. Pictures in panel B are derived from infections at MOI 0.5 (Calu-3 and T84 cells) or MOI 0.05 (Vero E6 cells).
   C) Comparison between the IC50 and CC50 values of cobicistat determined *in vitro* and the peak plasma levels detectable in mice and in after administration of a single dose of the drug. Determination of *in vitro* CC50 values is based on the data shown in (D).
   D) Uninfected cell lines of lung (Calu-3), gut (T84) and kidney (Vero E6) origin were left untreated or treated with serial dilutions of cobicistat. Forty-eight hours post-treatment cellular viability was measured by MTT assay. Data, expressed as mean ± SD of three independent experiments, were normalized to the untreated control and CC50 values were calculated by nonlinear regression.
**Figure 3****.** *Cobicistat decreases SARS-CoV-2 S-protein content and syncytia formation.*
   A,B). Screening of putative inhibitors of the enzymatic activity of 3CLₚᵣₒ. The activity of 3CLₚᵣₒ was measured by FRET assay and normalized over the untreated condition (A). Apart from cobicistat, compounds tested included HIV-1 protease inhibitors [nelfinavir, tipranavir] and compounds previously administered in clinical trials as SARS-CoV-2 therapeutics [darunavir, lopinavir], as well as two positive controls known to inhibit 3CLₚᵣₒ activity [MG132 and GC376]. EC50 values were calculated by nonlinear regression (B).
   C) Effect of cobicistat on the expression of S- and N-proteins in SARS-CoV-2 infected Vero E6 cells. Cells were infected at 0.5 MOI and left untreated or treated, two hours post-infection, with various concentrations of cobicistat, of the RdRP inhibitor remdesivir, or the 3CLₚᵣₒ inhibitor GC376. Cells were harvested 24 hours post-treatment and subjected to protein extraction and subsequent analysis by Western Blot. Expression of S- and N-proteins, and expression of the housekeeping protein actin-β, were detected using primary monoclonal antibodies followed by incubation with fluorescent-conjugated secondary antibodies and detection on a LI-COR Odyssey^{®} CLx instrument. Data are representative of three independent experiments.
   D,E) Effect of cobicistat on S-protein-mediated syncytia formation. Vero E6 cells were transfected with the SARS-CoV-2 S-protein and left untreated or treated with various concentrations of cobicistat or with sera isolated from convalescent SARS-CoV-2 patients (1:100 dilution). Syncytia formation was examined 24 hours post-transfection by immunofluorescence (IF) staining for DAPI and S-protein (D) and quantified as the number of cells forming syncytia (E). Data were analyzed using the nonparametric Kruskal-Wallis test followed by Dunn's post-test. Horizontal lines represent mean values. ** P < 0.01; **** P < 0.0001. Scale bar = 50µM.
**Figure 4****.** *Expression of the metabolic targets of cobicistat in uninfected and SARS-CoV-2 infected cell lines and primary human organoids.*
   A,B) The relative expression of CYP3A4/5 and P-gp was analyzed by qPCR in uninfected (A) and SARS-CoV-2 infected or mock infected (B) cells. Infections were carried out at MOI 0.5 for 48 hours. Raw data were used to calculate delta CT values (A), by using the *TBP* gene as housekeeping control. Fold changes, in infected over mock infected cells, were then calculated using the delta-delta CT method. Data in (B) are expressed as mean ± SD (N = 3).
**Figure 5**. *The combination of cobicistat and remdesivir synergistically inhibits SARS-CoV-2 activity.*
   A-F) Synergistic activity of cobicistat and remdesivir in inhibiting replication and cytopathic effects of SARS-CoV-2 in Vero E6 cells. Cells were infected at 0.5 MOI and left untreated or treated with the drugs at the indicated concentrations two hours-post infection. Forty Eight hours post-treatment: cells were fixed for immunofluorescence (IF) staining (A,B), supernatants were collected for qPCR (C-E) or cellular viability was analyzed (F). For IF detection, cells were stained with sera of SARS-CoV-2 patients and with the J2 antibody, which binds to double stranded RNA (Pape *et al.* 2020). The percentage of infected cells was determined by automatic acquisition of nine images per well (A), as described in Example 1. Scale bar = 100µM. Viral RNA in supernatants was detected by qPCR using an *in vitro* transcribed standard curve for absolute quantification (C-E) and data, expressed as mean ± SD, were transformed as Log₁₀ to restore normality and analyzed by one-way ANOVA, followed by Holm-Sidak's post-test (C). Cellular viability was measured by MTT assay (F).
   Isobologram analysis of synergism (D) (Chou 2010) was performed using the IC90 values for SARS-CoV-2 replication of cobicistat, remdesivir, or their combination, calculated by nonlinear regression. Synergism analyses of the inhibition of viral replication (E) or cytopathic effects (F) were performed with the SynergyFinder web-tool using the Zero Interaction Potency (ZIP) model based on inhibition values calculated as described in Example 1.
   G) Effect of the combination of cobicistat and remdesivir on SARS-CoV-2 RNA expression in supernatants of a primary human colon organoid. Treatment with cobicistat/remdesivir was performed two hours post-infection and supernatants were collected forty-eight hours post-treatment. Viral RNA was quantified as described for panel (C).
   For all panels N = 3 independent experiments, except for panel E (N = 2 independent experiments) and panel G (N = 2 replicates from one colon organoid donor). *** P < 0.001; ** P < 0.01; * P < 0.05.
**Figure 6****.** *Synergistic antiviral effect of cobicistat and remdesivir in the Vero E6 and T84 cell lines.*
   Effect of combined treatment of cobicistat and remdesivir on the viability of SARS-CoV-2 infected Vero E6 cells (A) and on viral replication (B,C) and inhibition of cytopathic effects (D) in T84 cells. Cells were infected at 0.5 MOI and left untreated or treated with the drugs at the indicated concentrations two hours-post infection. Forty Eight hours post-treatment: cells were fixed for crystal violet (A) or immunofluorescence (IF) (B) staining, supernatants were collected for qPCR (C), or cellular viability was analyzed (D). For IF detection, cells were stained with sera of SARS-CoV-2 patients (B). Viral RNA in supernatants was detected by qPCR using an *in vitro* transcribed standard curve for absolute quantification and data, expressed as mean ± SD, were analyzed by non-parametric Friedman test, followed by Dunn's post-test (C). Scale bar = 100µM. Cellular viability was measured by MTT assay and synergism analysis of the inhibition cytopathic effects was performed with the SynergyFinder web-tool using the Zero Interaction Potency (ZIP) model based on inhibition values calculated as described in the Methods section. For panels C,D N = 3 independent experiments. * P < 0.05.
**Figure 7** *Synergistic antiviral effect of cobicistat and chloroquine in the Calu-3 and T84 cell lines.*

Effect of combined treatment of cobicistat and remdesivir on the viability of SARS-CoV-2 infected Calu-3 (A) and T84 (B) cells. Cells were infected at 0.5 MOI and left untreated or treated with the drugs at the indicated concentrations two hours-post infection. Forty Eight hours post-treatment cellular viability was analyzed by MTT assay. Synergism analysis of the inhibition cytopathic effects was performed with the SynergyFinder web-tool using the Zero Interaction Potency (ZIP) model.

### EXAMPLES

### Example 1 Material and Methods

### 1. Virtual screening and molecular docking

Identification of potentially active SARS-CoV-2 inhibitors with desirable Absorption, Distribution, Metabolism, Excretion and Toxicity (ADME-Tox) properties, was performed by structure-based virtual screening (SBVS) of Drugbank V. 5.1.5(72) compounds targeting the three-dimensional structure of SARS-CoV-2 3CLₚᵣₒ. The analysis was focused on the substrate-binding site, which is located between domain I and II of 3CLₚᵣₒ. The binding site was identified using the publicly available 3D crystal structure [Protein Data Bank (PDB) ID: 6W63]. Structures of the previously described non-covalent protease inhibitor X77 (Andrianov *et al.,* 2020), natively co-crystallized with 3CLₚᵣₒ were used as a reference for the identification of binding-site coordinates and dimensions for the virtual screening workflow, as well as for the docking validation of positions generated from the screening.

Protein structure analysis and preparation for docking were performed using the Schrödinger protein preparation wizard (Schrödinger Inc). Missing hydrogen atoms were added, bond orders were corrected and unknown atom types were assigned. Protein side-chain amides were fixed using program default parameters and missing protein side chains were filled-in using the prime tool. All non-amino acid residues, including water molecules, were removed. Further, unrelated ligand molecules were removed and active ligand structures were extracted and isolated in separate files. Finally, the minimization of protein strain energy was achieved through restrained minimization options with default parameters. The centroids of extracted ligands were then used to identify the binding site with coordinates and dimensions extended for 20 Å stored as Glide grid file. Drug screening was performed using the Glide software (Friesner *et al.,* 2004). High throughput virtual screening (HTVS) was performed with the fastest search configurations. After post-docking minimization, the top-scoring tenth percentile of the output docked structures were subjected to the standard precision docking stage (SP). Then, active ligand structures were extracted and isolated in separate files. Finally, the top 10% scoring compounds were selected and retained only if their good scoring states were confirmed by Extra precision docking.

Remdesivir docking to CYP3A4, CYP3A5 and P-gp structures was performed to assess its capacity as a substrate/inhibitor for these proteins. CYP3A4, CYP3A5 and P-gp structures were collected from Protein Data Bank (PDB), IDs: 5VC0, 5VEU and 6QEE, respectively, and were subjected to the same preparation steps described above. Native inhibitors were used for identification of binding sites; the centroid of the known inhibitor Zosuquidar was used to identify the drug binding pocket of the P-gp protein structure. Further, co-crystallized Ritonavir was used for identification of the drug binding pocket in both CYP3A4/5. Receptor grids were generated for protein structures, for both CYP3A4 and CYP3A5. The heme iron of the Protoporphyrin ring was added as metal coordination constraint, allowing metal-ligand interaction in the subsequent docking steps. Docking was performed using flexible ligand conformer sampling allowing ring sampling with a 2.5 kcal/mol window. Retained poses for the initial docking phase were set to 5000 poses and only 800 best poses per ligand were selected for energy minimization. Finally, post-docking minimization was carried out for 10 poses per ligand with a 0.5 kcal/mol threshold for rejecting minimized poses.

### 2. Cell lines and primary human organoids

The following cell lines were used for infection and/or relative quantification of gene expression: Calu-3 (ATCC HTB-55), Caco-2 (ATCC HTB-37), T84 (ATCC CCL-24) and VeroE6 (ATCC CRL-1586). Primary organoids derived from human colon and ileum were seeded in 2D as described in (Stanifer *et al.* 2020). Culture conditions and susceptibility to SARS-CoV-2 infection have been previously described (Cortese *et al.* 2020; Stanifer *et al.* 2020).

### 3. Virus stock production and infection

Viral stocks used for infections were produced by passaging the BavPat1/2020 SARS-CoV-2 strain in Vero E6 cells and the infectious titer was estimated by plaque assay, as previously described. Infection experiments were conducted using 25,000 or 250,000 cells per well in 96 and 12 well plates, respectively. Cell lines were infected at 0.05 or 0.5 MOI in medium with low FCS content (2%). Colon organoids were infected in a 24-well plate using 60000 plaque forming units (PFU) per well. Two hours post-infection cells were washed twice in PBS and resuspended in complete medium.

### 4. Drug treatments

The following compounds were tested to determine their effects on 3CLₚᵣₒ activity, cytotoxicity or inhibition of SARS-CoV-2 replication: cobicistat (#sc-500831; Santa Cruz Biotechnology), remdesivir (#S78932; Selleckchem Chemicals), tipranavir (#sc-220260; Santa Cruz Biotechnology), nelfinavir mesylate hydrate (#PZ0013, Sigma-Aldrich), darunavir, lopinavir (both obtained through the AIDS Research and Reference Reagent Program, Division of AIDS, NIAID), MG-132 (#M8699; Sigma-Aldrich), GC376 (BPS Bioscience), Chloroquine (#C 6628, Sigma Aldrich).

### 5. RNA isolation and cDNA retrotranscription

RNA extraction was performed on cell lysates or supernatants using the NucleoSpin RNA, Mini kit for RNA purification (Macherey-Nagel, Düren, Germany) according to the manufacturer's instructions. The concentration of RNA extracted from cell lysates was measured using a P-class P 300 NanoPhotometer (Implen GmbH, Munich, Germany).

Retrotranscription to cDNA was performed with 500ng of intracellular RNA or 10µL of RNA from supernatants, using High-Capacity cDNA Reverse Transcription Kit (Applied Biosystems, Foster City, CA, USA) following the manufacturer's instructions.

### 6. SARS-CoV-2 RNA standard

For the preparation of a viral RNA standard to use in qPCR for quantification of viral copies in supernatants, SARS-CoV-2 N sequence was reverse transcribed from total RNA isolated from cells infected with the SARS-CoV-2 BavPat1 stain using Superscript 3 and specific primers (TTAGGCCTGAGTTGAGTCA, SEQ ID NO. 1). The resulting cDNA was amplified and cloned into the pJET1.2 plasmid. Ten µg of plasmid DNA was linearized by AdeI restriction enzyme digestion and DNA was purified using the NucleoSpin Gel and PCR Clean-up kit (Macherey-Nagel, Düren, Germany). For *in vitro* transcription T7 RNA polymerase was used as previously described (Fischl and Bartenschlager 2013). *In vitro* transcripts were purified by phenolchloroform extraction and resuspended in RNase-free water. RNA integrity was confirmed by agarose gel electrophoresis.

### 7. qPCR analysis

Gene and/or viral expression were analyzed by SYBR green qPCR using, for each reaction, 10µL of SsoFast^{™} EvaGreen^{®} Supermix (Bio-Rad Laboratories, Hercules, CA, USA), 500nM of forward and reverse primer (0.1µL each from 100µM stock), 8.8µL water and 1µL cDNA. The primers used are listed in Table 2. The qPCR reaction was performed on a CFX96/C1000 Touch qPCR system (Bio-Rad Laboratories, Hercules, CA, USA) using the following PCR program: polymerase activation/DNA denaturation 98°C for 3 min, followed by 45 cycles of denaturation at 98°C for 10s; annealing/extension at 60°C for 40s and a final extension step at the end of the program at 65°C for 30s. Gene expression data were normalized using the delta-delta CT method [2(-ΔΔ C(T)) method] (Livak and Schmittgen 2001), using the Tata-binding protein (*TBP*) gene as housekeeper control.

**Table 2 List of qPCR primers used in the study**

| ***Name*** | ***Sequence*** | ***Source*** | ***SEQ ID NO.*** |
|---|---|---|---|
| 2019-nCoV_N1-Forward | GAC CCC AAA ATC AGC GAA AT | ***(1)*** | 2 |
| 2019-nCoV_N1-Reverse | TCT GGT TAC TGC CAG TTG AAT CTG | | 3 |
| 2019-nCoV_N2-Forward | TTA CAA ACA TTG GCC GCA AA | ***(2)*** | 4 |
| 2019-nCoV_N2-Reverse | GCG CGA CAT TCC GAA GAA | | 5 |
| Hum Cyp3A4-Forward | TGA TGG CTC TCA TCC CAG AC | | 6 |
| Cyp3A4-Reverse | AGC CCC ACA CTT TTC CAT AC | | 7 |
| AGM Cyp3A4-Forward | TGA TGG ACC TCA TCC CAG AC | | 8 |
| Hum Cyp3A5-Forward | CGA CAA ACA AAA GCA CCG AC | | 9 |
| Hum Cyp3A5-Reverse | TTA TTG ACT GGG CTG CGA G | | 10 |
| AGM Cyp3A5-Forward | CGA CAA ACA AAA GCA CCG AG | | 11 |
| AGM Cyp3A5-Reverse | TAA TTG ATT GGG CCA CGA G | | 12 |
| P-gp (MDR1)-F | CCC ATC ATT GCA ATA GCA GG | Gao *et al.,* 2015 | 13 |
| P-gp (MDR1)-R | TGT TCA AAC TTC TGC TCC TGA | | 14 |
| TBP-F | CCA CTC ACA GAC TCT CAC AAC | Stanifer *et al.,* 2020 | 15 |
| TBP-R | CTG CGG TAC AAT CCC AGA ACT | | 16 |

| | | | |
|---|---|---|---|
| Hum = human AGM = african green monkey ***(1)*** https://www.cdc.gov/coronavirus/2019-ncov/lab/rt-pcr-panel-primer-probes.html ***(2)*** https://www.cdc.gov/coronavirus/2019-ncov/lab/rt-pcr-panel-primer-probes.html | | | |

### 8. Western blot

For Western blot experiments 0.5 × 10⁶ cells were lysed in a buffer (20 mM Tris-HCl, pH 7.4, 1 mM EDTA, 150 mM NaCl, 0.5% Nonidet P-40, 0.1% SDS, and 0.5% sodium deoxycholate supplemented with protease and phosphatase inhibitors (Sigma-Aldrich, Saint Louis, MI, USA). Lysates were boiled at 95°C for 10 min and sonicated for 5 min using a Bioruptor^{®} Plus sonication device (Diagenode, Liège, Belgium). Protein lysates were then run on a precast NuPAGE Bis-Tris 4-12% (Thermo Fisher Scientific, Waltham, MA, USA) SDS-PAGE at 100-120 V and transferred onto a nitrocellulose membrane (GE Healthcare, Little Chalfont, UK) for 2.5 h at 25 V using a Trans-Blot device for semi-dry transfer (Bio-Rad Laboratories, Hercules, CA, USA). Membranes were blocked using the LI-COR Intercept (PBS) Blocking Buffer (LI-COR Biosciences, Lincoln, NE, USA) for 1 h at RT and incubated overnight at 4°C with the following primary antibodies in blocking buffer with 0.2% Tween 20: α-β-actin (1:10,000), (Sigma-Aldrich, Saint Louis, MI, USA), α-SARS-CoV-2 spike protein [(rabbit; 1:1000) ab252690 Abcam], α-SARS-CoV-2 nucleocapsid [(mouse; 1:1000) AB_2827977, Sino Biological)], sera of SARS-CoV-2 positive individuals (1:200). Sera were collected as described in (Pape *et al.* 2020), following signing of informed consent by the donors, as well as ethical approval by Heidelberg University Hospital. After primary antibody incubation, membranes were washed three times with 0.1% PBS-Tween and incubated for 1 h with the following fluorescence-conjugated secondary antibodies: IRDye^{®} 800CW Goat anti-Human IgG, IRDye^{®} 800CW anti rabbit, IRDye^{®} 700CW anti mouse (LI-COR Biosciences, Lincoln, NE, USA). All secondary antibodies were diluted 1:15000 in blocking buffer + 0.2% Tween. After three washes with 0.1% PBS-Tween and one wash in PBS, fluorescence signals were acquired using a LI-COR Odyssey^{®} CLx instrument.

### 9. Re-processing of microarray and RNA-Seq data

Microarray gene expression data for CYP3A4/5 and P-gp in different anatomical tissues or cell lines were retrieved from Homo Sapiens Affymetrix Human Genome U133 Plus 2.0 Array dataset. Data were filtered by applying the criteria "Healthy sample status" and "No experimental treatment". From the initial list, tissues with sample size < 25 were filtered out. The anatomy search tool was used to plot Log2 expression ratios of the tested genes. Gene expression data in cell lines were retrieved, apart from the aforementioned microarray dataset, from the RNAseq "mRNA Gene Level Homo sapiens (ref: Ensembl 75)" dataset. The cell line condition filter was used to refine the analysis and include exclusively cell lines susceptible to SARS-CoV-2 infection (*i.e.* T84, Caco2, Calu-3 and A-549).

### 10. Cell viability

Cell viability was evaluated by (3- [4,5-dimethylthiazol-2-yl]-2,5 diphenyl tetrazolium bromide) (MTT) assay and by crystal violet staining as previously described (Shytaj *et al.* 2020; Feoktistova, Geserick, and Leverkus 2016). Briefly, the MTT assay was conducted using the CellTiter 96^{®} Non-Radioactive Cell Proliferation Assay (MTT) (Promega; Madison, WI, USA). Cells were plated in a 96-well plate at a concentration of 3 × 10⁶ cells/mL in 100 µl of medium. The MTT solution (15 µl) was added to each well and, after 2-4 h, the reaction was stopped by the addition of 100 µl of 10% SDS. Absorbance values were acquired using an Infinite 200 PRO (Tecan, Männedorf, Switzerland) multimode plate reader at 570 nm wavelength.

For the crystal violet staining, cells were fixed in 6% formaldehyde and incubated with 0.1% crystal violet for 15 mins. Unbound staining was then washed with H₂O and cells were imaged using a Nikon Eclipse Ts2-FL microscope.

### 11. 3CLₚᵣₒ FRET assay

The activity of 3CLₚᵣₒ was measured by FRET assay (BPS Bioscience, San Diego, CA, USA) according to the manufacturer's instructions and as previously described (Zhang *et al.* 2020). Briefly, serial dilutions of test compounds and known 3CLpro were incubated in a 384 well plate with the 3CLₚᵣₒ and its appropriate buffer, containing 0.5 M DTT. Wells without drugs or without 3CLₚᵣₒ were used as positive control of 3CLₚᵣₒ activity and blank control, respectively. After a 30 min incubation, the 3CLₚᵣₒ substrate was added to each well and the plate was stored for 4 hours in the dark. The fluorescence signal was acquired on an Infinite 200 PRO (Tecan, Männedorf, Switzerland) using an excitation wavelength of 360nm and a detection wavelength of 460nm. All Three separate experiments were conducted, with each experiment performed in duplicate. Relative 3CLₚᵣₒ was expressed as percentage of the positive control after subtraction of the blank.

### 12. Immunofluorescence and syncytia formation assay

Cells were seeded on iBIDI glass bottom 96 well plate and infected with SARS-CoV-2 strain BavPat1/2020 for 24-48 h at MOI 0.5. Cells were rinsed in PBS and fixed with 6% PFA, followed by permeabilization with 0.5% Triton X100 (Sigma) in PBS for 15 minutes. Cells were then subjected to a standard immunofluorescence staining protocol. Briefly, cells were blocked in 2% milk (Roth) in PBS and incubated with primary antibodies in PBS (anti ds-RNA mouse monoclonal J2 antibody (Scicons) 1:2000 and patient serum 1:250). Cells were washed twice in PBS 0.02% tween and incubated with secondary antibody in PBS (1: 1000 anti-mouse 568, Goat anti-human IgG-AlexaFluor 488 (Invitrogen, Thermofisher Scientific) for immunoglobulins detection in human serum and goat anti-mouse IgG-AlexaFluor 568 (Invitrogen, Thermofisher Scientific) for dsRNA detection). Nuclei were counterstained with Hoechst 33342 (Thermofisher Scientific, 0.002µg/ml in PBS) for 5 minutes, washed twice with PBS and stored at +4°C until imaging.

For syncytia formation assay, Vero E6 cells (0.2 x 10⁶ cells/well) were seeded on cover slips in a 12 well plate 24 h prior transfection. Cells were transfected using TransIT-2020 or TransIT-LT1 (Mirus) with 0.75 µg of pCDNA3.1(+)-SARS-CoV-2-S and 100 µl Opti-MEM per well. 2 h post transfection, cells were treated with cobicistat (final concentration of 1 µM, 5 µM and 10 µM), serum of patients (1:500 or 1:100) or DMSO (same concentration as in 10 µM cobicistat). 24 h post transfection, cells were washed twice with PBS and fixed in 4 % PFA for 20 min at room temperature. After another washing step, cells were permeabilized in 0.5 % Triton for 5 min at room temperature, washed and blocked in 3% lipid-free BSA in PBS-0.1% Tween-20 for 1 h at room temperature. After washing, cells were stained with the primary rabbit polyclonal anti-SARS-CoV-2 spike glycoprotein antibody (1:1000, Abcam) for 1 h at room temperature or overnight at 4 °C. After washing, cells were incubated with the secondary Alexa Fluor 488 goat anti-rabbit IgG antibody (1:500, Life Technologies) for 1 h at room temperature. After washing, cells were incubated with DAPI (1:1000, Sigma-Aldrich) for 1 min followed by washing with PBS and deionized water. Images were acquired with Nikon Eclipse Ts2-FL Inverted Microscope. Syncytia with three or more nuclei surrounded by the antibody staining were used for the quantification. The edges of the antibody staining were overdrawn with the polygon selection tool in ImageJ.

### 13. Microscopy and image analysis

Cells were imaged using motorized Nikon Ti2 widefield microscope or with Nikon/Andor (CSU W1) spinning disc using a Plan Apo lambda 20x/0.75 air objective and a back-illuminated EM-CCD camera (Andor iXon DU-888). JOBS module was used for automatic acquisition of 9 images per well. Images were acquired in 3 channels using the following excitation/emission settings: Ex 377/50, Em 447/60 (Hoechst); Ex 482/35, Em 536/40 (AlexaFluor 488); Ex 562/40, Em 624/40 (AlexaFluor 568). When spinning disc was used the excitation was performed with 405nm, 488nm and 561nm lasers.

Quantification of infected cells (expressed as percentage of total cells imaged per well) was performed using a custom-made macro in ImageJ. After camera offset subtraction and local background subtraction using the rolling ball algorithm, nuclei were segmented using automated local thresholding based on the Niblack method. Region of interest (represented by the ring (5 pixel wide) around the nucleus) was determined for each individual cell. Median signal intensity was measured in the region of interest in Alexa488 (serum) and Alexa568 (dsRNA) channels. Threshold for calling infected cells was manually determined for each individual experiment using the data from mock transfected cells. The same image analysis procedure and threshold was used for all wells within one experiment.

### 14. Statistical analysis

Data normality assumptions were tested by D'Agostino & Pearson normality test (for > 3). Multiple group comparisons were conducted by non-parametric Kruksal Wallis test, followed by Dunn's post-test, or by Two-Way ANOVA followed by Dunnet's post-test. Half maximal inhibitory (IC50) and cytotoxic (CC50) concentrations of the compounds tested were estimated by nonlinear regression using using relative inhibition values calculated according to the formula: % inhibition = 100 * (1 - (X - mock infected)/(infected untreated - mock infected)), where X is each given treatment condition. Data analysis was conducted using GraphPad Prism v6 (GraphPad Software, San Diego, CA, USA). Synergy scores were calculated using the SynergyFinder web-tool (Ianevski *et al.* 2020) using the Zero Interaction Potency (ZIP) model (Yadav *et al.* 2015).

The features disclosed in the foregoing description, in the claims and/or in the accompanying drawings may, both separately and in any combination thereof, be material for realizing the invention in diverse forms thereof.

### REFERENCES

Algaissi, Abdullah, Mohamed A. Alfaleh, Sharif Hala, Turki S. Abujamel, Sawsan S. Alamri, Sarah A. Almahboub, Khalid A. Alluhaybi, et al. 2020. "SARS-CoV-2 S1 and N-Based Serological Assays Reveal Rapid Seroconversion and Induction of Specific Antibody Response in COVID-19 Patients." Scientific Reports 10 (1): 16561.
Andrianov, Alexander M., Yuri V. Komoushenko, Anna D. Karpenko, Ivan P. Bosko, and Alexander V. Tuzikov. 2020. "Computational Discovery of Small Drug-like Compounds as Potential Inhibitors of SARS-CoV-2 Main Protease." Journal of Biomolecular Structure & Dynamics, July, 1-13.
Arribas, Jose Ramon, Jose Ramon Arribas, Arun J. Sanyal, Alex Soriano, Bum Sik Chin, Bum Sik Chin, Shirin Kalimuddin, et al. 2020. "557. Impact of Concomitant Hydroxychloroquine Use on Safety and Efficacy of Remdesivir in Moderate COVID-19 Patients." Open Forum Infectious Diseases. https://doi.org/10.1093/ofid/ofaa439.751.
Bartlett, J. A., R. DeMasi, J. Quinn, C. Moxham, and F. Rousseau. 2001. "Overview of the Effectiveness of Triple Combination Therapy in Antiretroviral-Naive HIV-1 Infected Adults." AIDS 15 (11): 1369-77.
Beigel, John H., Kay M. Tomashek, Lori E. Dodd, Aneesh K. Mehta, Barry S. Zingman, Andre C. Kalil, Elizabeth Hohmann, et al. 2020. "Remdesivir for the Treatment of Covid-19 - Final Report." The New England Journal of Medicine 383 (19): 1813-26.
Bradley, G., and V. Ling. 1994. "P-Glycoprotein, Multidrug Resistance and Tumor Progression." Cancer Metastasis Reviews 13 (2): 223-33.
Brandon, Esther F. A., Rolf W. Sparidans, Ronald D. van Ooijen, Irma Meijerman, Luis Lopez Lazaro, Ignacio Manzanares, Jos H. Beijnen, and Jan H. M. Schellens. 2007. "In Vitro Characterization of the Human Biotransformation Pathways of Aplidine, a Novel Marine AntiCancer Drug." Investigational New Drugs 25 (1): 9-19.
Cao, Bin, Yeming Wang, Danning Wen, Wen Liu, Jingli Wang, Guohui Fan, Lianguo Ruan, et al. 2020. "A Trial of Lopinavir-Ritonavir in Adults Hospitalized with Severe Covid-19." The New England Journal of Medicine 382 (19): 1787-99.
Chen, Jun, Lu Xia, Li Liu, Qingnian Xu, Yun Ling, Dan Huang, Wei Huang, et al. 2020. "Antiviral Activity and Safety of Darunavir/Cobicistat for the Treatment of COVID-19." Open Forum Infectious Diseases 7 (7): ofaa241.
Chien, Minchen, Thomas K. Anderson, Steffen Jockusch, Chuanjuan Tao, Xiaoxu Li, Shiv Kumar, James J. Russo, Robert N. Kirchdoerfer, and Jingyue Ju. 2020. "Nucleotide Analogues as Inhibitors of SARS-CoV-2 Polymerase, a Key Drug Target for COVID-19." Journal of Proteome Research 19 (11): 4690-97.
Chou, Ting-Chao. 2010. "Drug Combination Studies and Their Synergy Quantification Using the Chou-Talalay Method." Cancer Research 70 (2): 440-46.
Cortese, Mirko, Ji-Young Lee, Berati Cerikan, Christopher J. Neufeldt, Viola M. J. Oorschot, Sebastian Köhrer, Julian Hennies, et al. 2020. "Integrative Imaging Reveals SARS-CoV-2-Induced Reshaping of Subcellular Morphologies." Cell Host & Microbe 28 (6): 853-66.e5.
Cox, Robert M., Josef D. Wolf, and Richard K. Plemper. 2021. "Therapeutically Administered Ribonucleoside Analogue MK-4482/EIDD-2801 Blocks SARS-CoV-2 Transmission in Ferrets." Nature Microbiology 6 (1): 11-18.
Daina, Antoine, Olivier Michielin, and Vincent Zoete. 2017. "SwissADME: A Free Web Tool to Evaluate Pharmacokinetics, Drug-Likeness and Medicinal Chemistry Friendliness of Small Molecules." Scientific Reports 7 (March): 42717.
Deeks, Emma D. 2014. "Cobicistat: A Review of Its Use as a Pharmacokinetic Enhancer of Atazanavir and Darunavir in Patients with HIV-1 Infection." Drugs 74 (2): 195-206.
Fehr AR, Perlman S. Coronaviruses: An overview of their replication and pathogenesis. Coronaviruses: Methods and Protocols. 2015;1-23.
Feoktistova, Maria, Peter Geserick, and Martin Leverkus. 2016. "Crystal Violet Assay for Determining Viability of Cultured Cells." Cold Spring Harbor Protocols 2016 (4): db.prot087379.
Fischl, Wolfgang, and Ralf Bartenschlager. 2013. "High-Throughput Screening Using Dengue Virus Reporter Genomes." Methods in Molecular Biology 1030: 205-19.
Friesner, Richard A., Jay L. Banks, Robert B. Murphy, Thomas A. Halgren, Jasna J. Klicic, Daniel T. Mainz, Matthew P. Repasky, et al. 2004. "Glide: A New Approach for Rapid, Accurate Docking and Scoring. 1. Method and Assessment of Docking Accuracy." Journal of Medicinal Chemistry 47 (7): 1739-49.
Gao, B., Yang, F. M., Yu, Z. T., Li, R., Xie, F., Chen, J., Luo, H. J., & Zhang, J. C. (2015). Relationship between the expression of MDR1 in hepatocellular cancer and its biological behaviors. International journal of clinical and experimental pathology, 8(6), 6995-7001
Gulick, Roy M., and Charles Flexner. 2019. "Long-Acting HIV Drugs for Treatment and Prevention." Annual Review of Medicine. https://doi.org/10.1146/annurev-med-041217-013717.
Gupta, Manoj Kumar, Sarojamma Vemula, Ravindra Donde, Gayatri Gouda, Lambodar Behera, and Ramakrishna Vadde. 2020. "Approaches to Detect Inhibitors of the Human Severe Acute Respiratory Syndrome Coronavirus Envelope Protein Ion Channel." Journal of Biomolecular Structure & Dynamics, April, 1-11.
Guy, R. Kiplin, R. Kiplin Guy, Robert S. DiPaola, Frank Romanelli, and Rebecca E. Dutch. 2020. "Rapid Repurposing of Drugs for COVID-19." Science. https://doi.org/10.1126/science.abb9332.
Hoffmann, Markus, Hannah Kleine-Weber, and Stefan Pöhlmann. 2020. "A Multibasic Cleavage Site in the Spike Protein of SARS-CoV-2 Is Essential for Infection of Human Lung Cells." Molecular Cell 78 (4): 779-84.e5.
Hoffmann, Markus, Hannah Kleine-Weber, Simon Schroeder, Nadine Krüger, Tanja Herrler, Sandra Erichsen, Tobias S. Schiergens, et al. 2020. "SARS-CoV-2 Cell Entry Depends on ACE2 and TMPRSS2 and Is Blocked by a Clinically Proven Protease Inhibitor." Cell 181 (2): 271-80.e8.
Hu, Wen-Juan, Lu Chang, Ying Yang, Xin Wang, Yuan-Chao Xie, Jing-Shan Shen, Bo Tan, and Jia Liu. 2020. "Pharmacokinetics and Tissue Distribution of Remdesivir and Its Metabolites Nucleotide Monophosphate, Nucleotide Triphosphate, and Nucleoside in Mice." Acta Pharmacologica Sinica, October. https://doi.org/10.1038/s41401-020-00537-9.
lanevski, Aleksandr, Liye He, Tero Aittokallio, and Jing Tang. 2020. "SynergyFinder: A Web Application for Analyzing Drug Combination Dose-Response Matrix Data." Bioinformatics 36 (8): 2645.
Jácome, Rodrigo, José Alberto Campillo-Balderas, Samuel Ponce de León, Arturo Becerra, and Antonio Lazcano. 2020. "Sofosbuvir as a Potential Alternative to Treat the SARS-CoV-2 Epidemic." Scientific Reports 10 (1): 9294.
Jiang S, Hillyer C, Du L. Neutralizing Antibodies against SARS-CoV-2 and Other Human Coronaviruses. Trends in Immunology. 2020; July-August; 14(4): 407-412.
Jin, Zhenming, Xiaoyu Du, Yechun Xu, Yongqiang Deng, Meiqin Liu, Yao Zhao, Bing Zhang, et al. 2020. "Structure of M pro from SARS-CoV-2 and Discovery of Its Inhibitors." Nature 582 (7811): 289-93.
Jockusch, Steffen, Chuanjuan Tao, Xiaoxu Li, Thomas K. Anderson, Minchen Chien, Shiv Kumar, James J. Russo, Robert N. Kirchdoerfer, and Jingyue Ju. 2020. "A Library of Nucleotide Analogues Terminate RNA Synthesis Catalyzed by Polymerases of Coronaviruses That Cause SARS and COVID-19." Antiviral Research 180 (August): 104857.
Johnson, Raymond M., and Joseph M. Vinetz. 2020. "Dexamethasone in the Management of Covid - 19." BMJ. https://doi.org/10.1136/bmj.m2648.
Jorgensen, Sarah C. J., Razieh Kebriaei, and Linda D. Dresser. 2020. "Remdesivir: Review of Pharmacology, Pre-Clinical Data, and Emerging Clinical Experience for COVID-19." Pharmacotherapy 40 (7): 659-71.
Josephson, F. 2010. "Drug-Drug Interactions in the Treatment of HIV Infection: Focus on Pharmacokinetic Enhancement through CYP3A Inhibition." Journal of Internal Medicine 268 (6): 530-39.
Kakuda, Thomas N., Tom Van De Casteele, Romana Petrovic, Mark Neujens, Hiba Salih, Magda Opsomer, and Richard Mw Hoetelmans. 2014. "Bioequivalence of a Darunavir/cobicistat Fixed-Dose Combination Tablet versus Single Agents and Food Effect in Healthy Volunteers." Antiviral Therapy 19 (6): 597-606.
Kaptein, Suzanne J. F., Sofie Jacobs, Lana Langendries, Laura Seldeslachts, Sebastiaan ter Horst, Laurens Liesenborghs, Bart Hens, et al. 2020. "Favipiravir at High Doses Has Potent Antiviral Activity in SARS-CoV-2-infected Hamsters, Whereas Hydroxychloroquine Lacks Activity." Proceedings of the National Academy of Sciences. https://doi.org/10.1073/pnas.2014441117.
Kim, Kyoung-Ah, Ji-Young Park, Ji-Suk Lee, and Sabina Lim. 2003. "Cytochrome P450 2C8 and CYP3A4/5 Are Involved in Chloroquine Metabolism in Human Liver Microsomes." Archives of Pharmacal Research 26 (8): 631-37.
Klein, Steffen, Mirko Cortese, Sophie L. Winter, Moritz Wachsmuth-Melm, Christopher J. Neufeldt, Berati Cerikan, Megan L. Stanifer, Steeve Boulant, Ralf Bartenschlager, and Petr Chlanda. 2020. "SARS-CoV-2 Structure and Replication Characterized by in Situ Cryo-Electron Tomography." Nature Communications 11 (1): 5885.
Leegwater, Emiel, Anne Strik, Erik B. Wilms, Liesbeth B. E. Bosma, David M. Burger, Thomas H. Ottens, and Cees van Nieuwkoop. 2020. "Drug-Induced Liver Injury in a Patient With Coronavirus Disease 2019: Potential Interaction of Remdesivir With P-Glycoprotein Inhibitors." Clinical Infectious Diseases. https://doi.org/10.1093/cid/ciaa883.
Lepist, Eve-Irene, Truc K. Phan, Anupma Roy, Leah Tong, Kelly Maclennan, Bernard Murray, and Adrian S. Ray. 2012. "Cobicistat Boosts the Intestinal Absorption of Transport Substrates, Including HIV Protease Inhibitors and GS-7340, in Vitro." Antimicrobial Agents and Chemotherapy 56 (10): 5409-13.
Liu, Jia, Ruiyuan Cao, Mingyue Xu, Xi Wang, Huanyu Zhang, Hengrui Hu, Yufeng Li, Zhihong Hu, Wu Zhong, and Manli Wang. 2020. "Hydroxychloroquine, a Less Toxic Derivative of Chloroquine, Is Effective in Inhibiting SARS-CoV-2 Infection in Vitro." Cell Discovery 6 (March): 16.
Livak, K. J., and T. D. Schmittgen. 2001. "Analysis of Relative Gene Expression Data Using Real-Time Quantitative PCR and the 2(-Delta Delta C(T)) Method." Methods 25 (4): 402-8.
Ma, Chunlong, Michael Dominic Sacco, Brett Hurst, Julia Alma Townsend, Yanmei Hu, Tommy Szeto, Xiujun Zhang, et al. 2020. "Boceprevir, GC-376, and Calpain Inhibitors II, XII Inhibit SARS-CoV-2 Viral Replication by Targeting the Viral Main Protease." Cell Research 30 (8): 678-92.
Mahdi, Mohamed, János András Mótyán, Zsofia Ilona Szojka, Mária Golda, Márió Miczi, and Jozsef Tözsér. 2020. "Analysis of the Efficacy of HIV Protease Inhibitors against SARS-CoV-2's Main Protease." Virology Journal 17 (1): 190.
Maier, James A., Carmenza Martinez, Koushik Kasavajhala, Lauren Wickstrom, Kevin E. Hauser, and Carlos Simmerling. 2015. "ff14SB: Improving the Accuracy of Protein Side Chain and Backbone Parameters from ff99SB." Journal of Chemical Theory and Computation 11 (8): 3696-3713.
Mathias, A. A., P. German, B. P. Murray, L. Wei, A. Jain, S. West, D. Warren, J. Hui, and B. P. Kearney. 2010. "Pharmacokinetics and Pharmacodynamics of GS-9350: A Novel Pharmacokinetic Enhancer without Anti-HIV Activity." Clinical Pharmacology and Therapeutics 87 (3): 322-29.
Miller, Bill R., 3rd, T. Dwight McGee Jr, Jason M. Swails, Nadine Homeyer, Holger Gohlke, and Adrian E. Roitberg. 2012. "MMPBSA.py: An Efficient Program for End-State Free Energy Calculations." Journal of Chemical Theory and Computation 8 (9): 3314-21.
Mulangu, Sabue, Lori E. Dodd, Richard T. Davey Jr, Olivier Tshiani Mbaya, Michael Proschan, Daniel Mukadi, Mariano Lusakibanza Manzo, et al. 2019. "A Randomized, Controlled Trial of Ebola Virus Disease Therapeutics." The New England Journal of Medicine 381 (24): 2293-2303.
Naggie, Susanna, and Andrew J. Muir. 2017. "Oral Combination Therapies for Hepatitis C Virus Infection: Successes, Challenges, and Unmet Needs." Annual Review of Medicine 68 (January): 345-58.
Ou, Xiuyuan, Yan Liu, Xiaobo Lei, Pei Li, Dan Mi, Lili Ren, Li Guo, et al. 2020. "Characterization of Spike Glycoprotein of SARS-CoV-2 on Virus Entry and Its Immune Cross-Reactivity with SARS-CoV." Nature Communications 11 (1): 1620.
Pal M, Berhanu G, Desalegn C, Kandi V. Severe Acute Respiratory Syndrome Coronavirus-2 (SARS-CoV-2): An Update. Cureus. 2020; Mar; 12(3): e7423.
Pape, Constantin, Roman Remme, Adrian Wolny, Sylvia Olberg, Steffen Wolf, Lorenzo Cerrone, Mirko Cortese, et al. 2020. "Microscopy-based Assay for Semi-quantitative Detection of SARS-CoV-2 Specific Antibodies in Human Sera." BioEssays. https://doi.org/10.1002/bies.202000257.
Pawlotsky, Jean-Michel, Jordan J. Feld, Stefan Zeuzem, and Jay H. Hoofnagle. 2015. "From Non-A, Non-B Hepatitis to Hepatitis C Virus Cure." Journal of Hepatology 62 (1 Suppl): S87-99.
Pires, Douglas E. V., Tom L. Blundell, and David B. Ascher. 2015. "pkCSM: Predicting Small-Molecule Pharmacokinetic and Toxicity Properties Using Graph-Based Signatures." Journal of Medicinal Chemistry 58 (9): 4066-72.
Pushpakom, Sudeep, Francesco Iorio, Patrick A. Eyers, K. Jane Escott, Shirley Hopper, Andrew Wells, Andrew Doig, et al. 2019. "Drug Repurposing: Progress, Challenges and Recommendations." Nature Reviews. Drug Discovery 18 (1): 41-58.
Razzaghi-Asl, Nima, Ahmad Ebadi, Sara Shahabipour, and Danial Gholamin. 2020. "Identification of a Potential SARS-CoV2 Inhibitor via Molecular Dynamics Simulations and Amino Acid Decomposition Analysis." Journal of Biomolecular Structure & Dynamics, July, 1 - 16.
RECOVERY Collaborative Group, Peter Horby, Wei Shen Lim, Jonathan R. Emberson, Marion Mafham, Jennifer L. Bell, Louise Linsell, et al. 2020. "Dexamethasone in Hospitalized Patients with Covid-19 - Preliminary Report." The New England Journal of Medicine, July. https://doi.org/10.1056/NEJMoa2021436.
RECOVERY Collaborative Group, Peter Horby, Marion Mafham, Louise Linsell, Jennifer L. Bell, Natalie Staplin, Jonathan R. Emberson, et al. 2020. "Effect of Hydroxychloroquine in Hospitalized Patients with Covid-19." The New England Journal of Medicine 383 (21): 2030-40.
Richter, Oliver von, Oliver Burk, Martin F. Fromm, Klaus P. Thon, Michel Eichelbaum, and Kari T. Kivistö. 2004. "Cytochrome P450 3A4 and P-Glycoprotein Expression in Human Small Intestinal Enterocytes and Hepatocytes: A Comparative Analysis in Paired Tissue Specimens." Clinical Pharmacology and Therapeutics 75 (3): 172-83.
Roe, Daniel R., and Thomas E. Cheatham 3rd. 2013. "PTRAJ and CPPTRAJ: Software for Processing and Analysis of Molecular Dynamics Trajectory Data." Journal of Chemical Theory and Computation 9 (7): 3084-95.
Sherman, Elizabeth M., Marylee V. Worley, Nathan R. Unger, Timothy P. Gauthier, and Jason J. Schafer. 2015. "Cobicistat: Review of a Pharmacokinetic Enhancer for HIV Infection." Clinical Therapeutics 37 (9): 1876-93.
Shytaj, Iart Luca, Bojana Lucic, Mattia Forcato, Carlotta Penzo, James Billingsley, Vibor Laketa, Steven Bosinger, et al. 2020. "Alterations of Redox and Iron Metabolism Accompany the Development of HIV Latency." The EMBO Journal 39 (9): e102209.
Siegel, Dustin, Hon C. Hui, Edward Doerffler, Michael O. Clarke, Kwon Chun, Lijun Zhang, Sean Neville, et al. 2017. "Discovery and Synthesis of a Phosphoramidate Prodrug of a Pyrrolo[2,1-F][triazin-4-Amino] Adenine C-Nucleoside (GS-5734) for the Treatment of Ebola and Emerging Viruses." Journal of Medicinal Chemistry. https://doi.org/10.1021/acs.jmedchem.6b01594.
Stanifer, Megan L., Carmon Kee, Mirko Cortese, Camila Metz Zumaran, Sergio Triana, Markus Mukenhim, Hans-Georg Kraeusslich, Theodore Alexandrov, Ralf Bartenschlager, and Steeve Boulant. 2020. "Critical Role of Type III Interferon in Controlling SARS-CoV-2 Infection in Human Intestinal Epithelial Cells." Cell Reports 32 (1): 107863.
Tian, Siyang, Yannick Djoumbou-Feunang, Russell Greiner, and David S. Wishart. 2018. "CypReact: A Software Tool for in Silico Reactant Prediction for Human Cytochrome P450 Enzymes." Journal of Chemical Information and Modeling 58 (6): 1282-91.
V'kovski, Philip, Annika Kratzel, Silvio Steiner, Hanspeter Stalder, and Volker Thiel. 2020. "Coronavirus Biology and Replication: Implications for SARS-CoV-2." Nature Reviews. Microbiology, October. https://doi.org/10.1038/s41579-020-00468-6.
von Hentig N. Clinical use of cobicistat as a pharmacoenhancer of human immunodeficiency virus therapy. HIV/AIDS - Research and Palliative Care. 2015;8:1-16.
Wang, Junmei, Romain M. Wolf, James W. Caldwell, Peter A. Kollman, and David A. Case. 2004. "Development and Testing of a General Amber Force Field." Journal of Computational Chemistry 25 (9): 1157-74.
Wang, Manli, Ruiyuan Cao, Leike Zhang, Xinglou Yang, Jia Liu, Mingyue Xu, Zhengli Shi, Zhihong Hu, Wu Zhong, and Gengfu Xiao. 2020. "Remdesivir and Chloroquine Effectively Inhibit the Recently Emerged Novel Coronavirus (2019-nCoV) in Vitro." Cell Research 30 (3): 269-71.
Wang, Yeming, Dingyu Zhang, Guanhua Du, Ronghui Du, Jianping Zhao, Yang Jin, Shouzhi Fu, et al. 2020. "Remdesivir in Adults with Severe COVID-19: A Randomised, Double-Blind, Placebo-Controlled, Multicentre Trial." The Lancet 395 (10236): 1569-78.
Waterschoot, R. A. B. van, R. ter Heine, E. Wagenaar, C. M. M. van der Kruijssen, R. W. Rooswinkel, A. D. R. Huitema, J. H. Beijnen, and A. H. Schinkel. 2010. "Effects of Cytochrome P450 3A (CYP3A) and the Drug Transporters P-Glycoprotein (MDR1/ABCB1) and MRP2 (ABCC2) on the Pharmacokinetics of Lopinavir." British Journal of Pharmacology 160 (5): 1224-33.
Wessler, Jeffrey D., Laura T. Grip, Jeanne Mendell, and Robert P. Giugliano. 2013. "The P-Glycoprotein Transport System and Cardiovascular Drugs." Journal of the American College of Cardiology 61 (25): 2495-2502.
White, Kris M., Romel Rosales, Soner Yildiz, Thomas Kehrer, Lisa Miorin, Elena Moreno, Sonia Jangra, et al. 2021. "Plitidepsin Has Potent Preclinical Efficacy against SARS-CoV-2 by Targeting the Host Protein eEF1A." Science, January. https://doi.org/10.1126/science.abf4058.
Xiu, Siyu, Alexej Dick, Han Ju, Sako Mirzaie, Fatemeh Abdi, Simon Cocklin, Peng Zhan, and Xinyong Liu. 2020. "Inhibitors of SARS-CoV-2 Entry: Current and Future Opportunities." Journal of Medicinal Chemistry 63 (21): 12256-74.
Xu L, Liu H, Murray BP, Callebaut C, Lee MS, Hong A, Strickley RG, Tsai LK, Stray KM, Wang Y, Rhodes GR, Desai MC. Cobicistat (GS-9350): A Potent and Selective Inhibitor of Human CYP3A as a Novel Pharmacoenhancer. ACS Med Chem Lett. 2010 Aug 12; 1(5): 209-213.
Yadav, Bhagwan, Krister Wennerberg, Tero Aittokallio, and Jing Tang. 2015. "Searching for Drug Synergy in Complex Dose-Response Landscapes Using an Interaction Potency Model." Computational and Structural Biotechnology Journal 13 (September): 504-13.
Yamamoto, Norio, Shutoku Matsuyama, Tyuji Hoshino, and Naoki Yamamoto. n.d. "Nelfinavir Inhibits Replication of Severe Acute Respiratory Syndrome Coronavirus 2 in Vitro." https://doi.org/10.1101/2020.04.06.026476.
Yamamoto, Norio, Rongge Yang, Yoshiyuki Yoshinaka, Shinji Amari, Tatsuya Nakano, Jindrich Cinatl, Holger Rabenau, et al. 2004. "HIV Protease Inhibitor Nelfinavir Inhibits Replication of SARS-Associated Coronavirus." Biochemical and Biophysical Research Communications 318 (3): 719-25.
Yang, Katherine. 2020. "What Do We Know About Remdesivir Drug Interactions?" Clinical and Translational Science 13 (5): 842-44.
Zanger, Ulrich M., and Matthias Schwab. 2013. "Cytochrome P450 Enzymes in Drug Metabolism: Regulation of Gene Expression, Enzyme Activities, and Impact of Genetic Variation." Pharmacology & Therapeutics 138 (1): 103-41.
Zhang, Linlin, Daizong Lin, Xinyuanyuan Sun, Ute Curth, Christian Drosten, Lucie Sauerhering, Stephan Becker, Katharina Rox, and Rolf Hilgenfeld. 2020. "Crystal Structure of SARS-CoV-2 Main Protease Provides a Basis for Design of Improved α-Ketoamide Inhibitors." Science 368 (6489): 409-12.

## Claims

1. Cobicistat for use in the prophylaxis and/or treatment of a severe acute respiratory syndrome coronavirus type 2 (SARS-CoV-2) infection, a severe acute respiratory syndrome coronavirus (SARS-CoV) infection, and/or a Middle East respiratory syndrome coronavirus (MERS-CoV) infection,
wherein cobicistat is administered orally at a daily dosage of 300 to 1,000 mg.

2. Cobicistat for use according to claim 1, in the prophylaxis and/or treatment of Coronavirus disease 2019 (COVID-19).

3. Cobicistat for use according to claim 1 or 2, in combination with one or more further drug.

4. Cobicistat for use according to claim 3, wherein the one or more further drug is an antiviral agent, preferably remdesivir, chloroquine, hydroxychloroquine, molnupiravir, or favipiravir.

5. Cobicistat for use according to claim 3 or 4, wherein the one or more further drug is an HIV protease inhibitor, preferably tipranavir, nelfinavir, lopinavir, or atazanavir.

6. Cobicistat for use according to claim 3 or 4, wherein the one or more further drug is plitidepsin,
and/or an anti-inflammatory glucocorticoid,
such as dexamethasone, prednisone, methylprednisolone, or hydrocortisone,
and/or a januskinase (JAK) inhibitor,
such as baricitinib, ruxolitinib, or upadacitinib,
and/or a palmitoyl protein thioesterase 1 *(*PPT1*)* inhibitor,
such as GNS561,
and/or a monoclonal antibody targeting viral replication or host inflammation,
such as tocilizumab.

7. Cobicistat for use according to claim 3 or 4, wherein the further drug is remdesivir.

8. Cobicistat for use according to claim 3 or 4, wherein the one or more further drug is remdesivir, tipranavir, chloroquine, hydroxychloroquine, molnupiravir, favipiravir, nelfinavir, lopinavir, atazanavir, plitidepsin, dexamethasone, baricitinib, or GNS561.

9. Cobicistat for use according to any one of claims 3, 4 or 7, in combination with remdesivir in combination with one or more further drug, preferably with an HIV protease inhibitor, more preferably tipranavir, nelfinavir, lopinavir, or atazanavir.

10. Cobicistat for use according to any one of claims 3, 4 or 7, in combination with chloroquine in combination with one or more further drug, preferably with an HIV protease inhibitor, more preferably tipranavir, nelfinavir, lopinavir, or atazanavir.

## Patentansprüche

1. Cobicistat zur Verwendung bei der Prophylaxe und/oder Behandlung einer schweren akuten Atemwegssyndrom-Coronavirus Typ 2 (SARS-CoV-2) Infektion, einer schweren akuten Atemwegssyndrom-Coronavirus (SARS-CoV) Infektion, und/oder einer Nahost-Atemwegssyndrom-Coronavirus (MERS-CoV) Infektion,
wobei Cobicistat in einer Tagesdosis von 300 bis 1.000 mg oral verabreicht wird.

2. Cobicistat zur Verwendung nach Anspruch 1, bei der Prophylaxe und/oder Behandlung der Coronavirus-Krankheit 2019 (COVID-19).

3. Cobicistat zur Verwendung nach Anspruch 1 oder 2, in Kombination mit einem oder mehreren zusätzlichen Arzneimitteln.

4. Cobicistat zur Verwendung nach Anspruch 3, wobei das eine oder mehrere zusätzliche Arzneimittel ein antivirales Mittel ist, vorzugsweise Remdesivir, Chloroquin, Hydroxychloroquin, Molnupiravir, oder Favipiravir.

5. Cobicistat zur Verwendung nach Anspruch 3 oder 4, wobei das eine oder mehrere zusätzliche Arzneimittel ein HIV-Proteaseinhibitor ist, vorzugsweise Tipranavir, Nelfinavir, Lopinavir, oder Atazanavir.

6. Cobicistat zur Verwendung nach Anspruch 3 oder 4, wobei das eine oder mehrere zusätzliche Arzneimittel Plitidepsin ist,
und/oder ein entzündungshemmendes Glukokortikoid,
wie beispielsweise Dexamethason, Prednison, Methylprednisolon, oder Hydrocortison,
und/oder ein Januskinase (JAK)-Inhibitor,
wie beispielsweise Baricitinib, Ruxolitinib, oder Upadacitinib,
und/oder ein Palmitoylprotein-Thioesterase 1 *(*PPT1*)*-Inhibitor,
wie beispielsweise GNS561,
und/oder ein monoklonaler Antikörper, der auf die virale Replikation oder die Entzündung des Wirts abzielt,
wie beispielsweise Tocilizumab.

7. Cobicistat zur Verwendung nach Anspruch 3 oder 4, wobei das zusätzliche Arzneimittel Remdesivir ist.

8. Cobicistat zur Verwendung nach Anspruch 3 oder 4, wobei das eine oder mehrere zusätzliche Arzneimittel Remdesivir, Tipranavir, Chloroquin, Hydroxychloroquin, Molnupiravir, Favipiravir, Nelfinavir, Lopinavir, Atazanavir, Plitidepsin, Dexamethason, Baricitinib, oder GNS561 ist.

9. Cobicistat zur Verwendung nach einem der Ansprüche 3, 4 oder 7, in Kombination mit Remdesivir in Kombination mit einem oder mehreren zusätzlichen Arzneimitteln, vorzugsweise mit einem HIV-Proteaseinhibitor, bevorzugter Tipranavir, Nelfinavir, Lopinavir, oder Atazanavir.

10. Cobicistat zur Verwendung nach einem der Ansprüche 3, 4 oder 7, in Kombination mit Chloroquin in Kombination mit einem oder mehreren zusätzlichen Arzneimitteln, vorzugsweise mit einem HIV-Proteaseinhibitor, bevorzugter Tipranavir, Nelfinavir, Lopinavir, oder Atazanavir.

## Revendications

1. Cobicistat destiné à être utilisé dans la prophylaxie et/ou le traitement d'une infection par le coronavirus du syndrome respiratoire aigu sévère de type 2 (SARS-CoV-2), d'une infection par le coronavirus du syndrome respiratoire aigu sévère (SARS-CoV) et/ou d'une infection par le coronavirus du syndrome respiratoire du Moyen-Orient (MERS-CoV), dans lequel le cobicistat est administré par voie orale à une dose quotidienne de 300 à 1 000 mg.

2. Cobicistat destiné à être utilisé selon la revendication 1, dans la prophylaxie et/ou le traitement de la maladie à coronavirus 2019 (COVID-19).

3. Cobicistat destiné à être utilisé selon la revendication 1 ou 2, en combinaison avec un ou plusieurs médicaments supplémentaires.

4. Cobicistat destiné à être utilisé selon la revendication 3, dans lequel le un ou plusieurs médicaments supplémentaires est un agent antiviral, de préférence le remdésivir, la chloroquine, l'hydroxychloroquine, le molnupiravir, ou le favipiravir.

5. Cobicistat destiné à être utilisé selon la revendication 3 ou 4, dans lequel le un ou plusieurs médicaments supplémentaires est un inhibiteur de la protéase du VIH, de manière préférée le tipranavir, le nelfinavir, le lopinavir ou l'atazanavir.

6. Cobicistat destiné à être utilisé selon la revendication 3 ou 4, dans lequel le un ou plusieurs médicaments supplémentaires est la plitidepsine,
et/ou un glucocorticoïde anti-inflammatoire,
tel que la dexaméthasone, la prednisone, la méthylprednisolone ou l'hydrocortisone
et/ou un inhibiteur des Janus kinases (JAK),
tel que le baricitinib, le ruxolitinib ou l'upadacitinib,
et/ou un inhibiteur de la palmitoyl-protéine thioestérase 1 (PPT1),
tel que le GNS561,
et/ou un anticorps monoclonal ciblant la réplication virale ou une inflammation chez l'hôte,
tel que le tocilizumab.

7. Cobicistat destiné à être utilisé selon la revendication 3 ou 4, dans lequel le médicament supplémentaire est le remdésivir.

8. Cobicistat destiné à être utilisé selon la revendication 3 ou 4, dans lequel le un ou plusieurs médicaments supplémentaires est le remdésivir, le tipranavir, la chloroquine, l'hydroxychloroquine, le molnupiravir, le favipiravir, le nelfinavir, le lopinavir, l'atazanavir, la plitidepsine, la dexaméthasone, le baricitinib ou le GNS561.

9. Cobicistat destiné à être utilisé selon l'une quelconque des revendications 3, 4 ou 7 en combinaison avec le remdésivir en combinaison avec un ou plusieurs médicaments supplémentaires, de préférence avec un inhibiteur de la protéase du VIH, de manière davantage préférée le tipranavir, le nelfinavir, le lopinavir ou l'atazanavir.

10. Cobicistat destiné à être utilisé selon l'une quelconque des revendications 3, 4 ou 7, en combinaison avec la chloroquine en combinaison avec un ou plusieurs médicaments supplémentaires, de préférence avec un inhibiteur de la protéase du VIH, de manière davantage préférée le tipranavir, le nelfinavir, le lopinavir ou l'atazanavir.
